(19) Europäisches Patentamt
European Patent Office
Office européen des brevets

(11) EP 1 991 235 B1

(12) **FASCICULE DE BREVET EUROPEEN**

(45) Date de publication et mention de la délivrance du brevet:
**02.11.2016 Bulletin 2016/44**

(21) Numéro de dépôt: **07731035.7**

(22) Date de dépôt: **23.02.2007**

(51) Int Cl.:
*A61P 43/00* (2006.01) *C07C 251/44* (2006.01)
*A61K 31/15* (2006.01) *A61K 31/223* (2006.01)
*A61K 31/496* (2006.01) *A61K 9/00* (2006.01)
*A61K 31/19* (2006.01) *A61K 31/122* (2006.01)
*A61K 31/16* (2006.01)

(86) Numéro de dépôt international:
**PCT/FR2007/000330**

(87) Numéro de publication internationale:
**WO 2007/101925 (13.09.2007 Gazette 2007/37)**

(54) **UTILISATION DE DÉRIVÉS DU 3,5-SÉCO-4-NOR-CHOLESTANE POUR L'OBTENTION D'UN MÉDICAMENT CYTOPROTECTEUR**

VERWENDUNG VON 3,5-SECO-4-NOR-CHOLESTAN-DERIVATEN ZUR HERSTELLUNG EINES ZYTOPROTEKTIVEN ARZNEIMITTELS

USE OF 3,5-SECO-4-NORCHOLESTANE DERIVATIVES FOR OBTAINING A CYTOPROTECTIVE MEDICAMENT

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **09.03.2006 FR 0602091**

(43) Date de publication de la demande:
**19.11.2008 Bulletin 2008/47**

(73) Titulaire: **Trophos**
**13288 Marseille Cedex 9 (FR)**

(72) Inventeurs:
- **PRUSS, Rebecca**
**F-13260 Cassis (FR)**
- **BUISSON, Bruno**
**F-13001 Marseille (FR)**
- **BORDET, Thierry**
**F-13009 Marseille (FR)**

(74) Mandataire: **Santarelli**
**146, rue Paradis**
**13294 Marseille Cedex 6 (FR)**

(56) Documents cités:
**EP-A1- 0 572 165**
**WO-A-2004/106370**
**WO-A-2006/027454**
**WO-A2-2005/007192**
**US-A- 3 763 228**

- **SMITH, RODNEY F. ET AL: "Steroid lysis of protoplasts and effects of stabilizers and steroid antagonists" APPLIED MICROBIOLOGY , 13(5), 706-12 CODEN: APMBAY; ISSN: 0003-6919, 1965, XP009074463**
- **SMITH ET AL: "RELATION OF SURFACTANT PROPERTIES OF SOME SYNTHETIC STEROIDS TO BACTERICIDAL ACTION" JOURNAL OF PHARMACEUTICAL SCIENCES, AMERICAN PHARMACEUTICAL ASSOCIATION. WASHINGTON, US, vol. 53, no. 10, 1964, pages 1214-1216, XP009074462 ISSN: 0022-3549**
- **SMITH R F ET AL: "EFFECTS OF PROTEIN, LIPIDS, AND SURFACTANTS ON THE ANTIMICROBIAL ACTIVITY OF SYNTHETIC STEROIDS." APPLIED MICROBIOLOGY NOV 1963, vol. 11, novembre 1963 (1963-11), pages 542-544, XP002436906 ISSN: 0003-6919**
- **LINGHAM R B ET AL: "Clavaric acid and steroidal analogues as ras- and FPP-directed inhibitors of human farnesyl-protein transferase" JOURNAL OF MEDICINAL CHEMISTRY, AMERICAN CHEMICAL SOCIETY. WASHINGTON, US, vol. 41, no. 23, 5 novembre 1998 (1998-11-05), pages 4492-4501, XP002286689 ISSN: 0022-2623**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

(Cont. page suivante)

- **PENG H ET AL: "Steroidal derived acids as inhibitors of human Cdc25A protein phosphatase." BIOORGANIC & MEDICINAL CHEMISTRY. FEB 2000, vol. 8, no. 2, février 2000 (2000-02), pages 299-306, XP002405229 ISSN: 0968-0896**
- **EDWARD J T ET AL: "RING CHAIN TAUTOMERISM OF 5 OXO-3 5-SECO-A NORCHOLESTAN-3-OL" CANADIAN JOURNAL OF CHEMISTRY, vol. 51, no. 10, 1973, pages 1610-1616, XP009074436 ISSN: 0008-4042**
- **RODEWALD, W. J. ET AL: "Azasteroidal alkaloids. Synthesis of A-nor-B-homo-5-azacholestane" BULLETIN DE L'ACADEMIE POLONAISE DES SCIENCES, SERIE DES SCIENCES CHIMIQUES , 11(8), 437-41 CODEN: BAPCAQ; ISSN: 0001-4095, 1963, XP009074061 cité dans la demande**


Remarques:

Le dossier contient des informations techniques présentées postérieurement au dépôt de la demande et ne figurant pas dans le présent fascicule.

## Description

La présente invention concerne l'utilisation de dérivés du 3,5-seco-4-nor-cholestane pour l'obtention d'un médicament cytoprotecteur, à l'exception d'un médicament neuroprotecteur.

Les processus dégénératifs cellulaires sont caractérisés par le dysfonctionnement des cellules entraînant souvent des activités cellulaires indésirables et la mort cellulaire.

Les cellules ont développé des mécanismes d'adaptation, en réponse au stress, qui allongent leur durée de vie ou retardent ou empêchent la mort cellulaire (mécanismes cytoprotecteurs).

Cependant, ces mécanismes cytoprotecteurs sont parfois insuffisants, inadéquats, ou induits trop tard pour être efficaces et les cellules meurent. Il peut donc s'avérer intéressant de disposer de nouveaux médicaments, cytoprotecteurs, qui favoriseraient la cytoprotection. C'est un des buts de la présente invention.

Le terme « cytoprotecteur» fait référence à la capacité d'agents, naturels ou non, à protéger une cellule contre la mort cellulaire, particulièrement la mort cellulaire pathologique, et/ou contre les dysfonctionnements cellulaires conduisant à la mort cellulaire. Ces dysfonctionnements cellulaires peuvent être par exemple d'origine mitochondriale, comme une réduction de la capacité à générer de l'ATP, une incapacité à capter et/ou retenir le calcium, ou la génération de radicaux libres.

Parmi les mécanismes principaux de mort cellulaire, on distingue essentiellement la nécrose, l'apoptose et la nécroptose.

La nécrose est une mort cellulaire dite "accidentelle" qui survient lors d'un dommage tissulaire. C'est la membrane plasmique de la cellule qui est la plus touchée, entraînant une modification de l'homéostasie de la cellule. Les cellules vont se gorger d'eau au point que cela va entraîner la lyse de leur membrane plasmique. Cette lyse cellulaire conduit au largage dans le milieu environnant du contenu cytoplasmique. La nécrose est à l'origine du processus inflammatoire.

La nécrose peut toucher un ensemble de cellules ou un tissu alors que les autres parties de voisinage restent vivantes. La transformation qui en résulte est une mortification des cellules ou des tissus.

Autrement dit, la nécrose se définit par des modifications morphologiques survenant lorsqu'une cellule arrive en fin de vie à la suite d'événements tels qu'un traumatisme important comme un arrêt ou une diminution de la circulation sanguine au niveau d'un organe, l'hyperthermie (élévation importante de la température), une intoxication par un produit chimique, un choc physique, etc... Une des nécroses les plus connues est celle du myocarde lors de l'infarctus (arrêt d'apport circulatoire au niveau du muscle cardiaque) due à une oblitération (obstruction) d'une artère coronaire.

L'apoptose fait partie intégrante de la physiologie normale d'un organisme. C'est une forme physiologique de mort cellulaire hautement régulée et elle est nécessaire à la survie des organismes multicellulaires. L'apoptose est un processus qui joue un rôle primordial au cours de l'embryogenèse.

Les cellules en apoptose ou apoptotiques vont s'isoler des autres cellules. L'apoptose implique habituellement des cellules individuelles dans un tissu et ne provoque pas l'inflammation. L'un des points morphologiques caractéristiques de l'apoptose est l'importante condensation à la fois du noyau et du cytoplasme ce qui induit une diminution significative du volume cellulaire. Le noyau se fragmente ensuite, chaque fragment est entouré d'une double enveloppe. Des corps apoptotiques (éléments cytoplasmiques et nucléaires) sont ensuite libérés et vont être absorbés par phagocytose par les cellules voisines.

L'apoptose peut être induite de différentes façons. Par exemple, une radiation, la présence d'un composé chimique ou d'une hormone sont des stimuli susceptibles d'induire une cascade d'événements apoptotiques dans la cellule. Des signaux intracellulaires comme une mitose incomplète ou un dommage à l'ADN peuvent aussi induire l'apoptose.

L'apoptose intervient aussi après l'action d'un génotoxique ou au cours d'une maladie. Certaines pathologies sont caractérisées par une apoptose anormale, entraînant la perte de certaines populations cellulaires, comme par exemple l'hépatotoxicité, les rétinopathies, la cardiotoxicité.

On distingue donc l'apoptose physiologique et l'apoptose pathologique. L'invention s'adresse essentiellement à l'apoptose pathologique.

Il existe d'autres mécanismes de mort cellulaire, comme par exemple la nécroptose, qui présente des caractéristiques de la nécrose et de l'apoptose. Une cellule mourrant par nécroptose présente des caractéristiques similaires à celles d'une cellule mourrant par nécrose, mais les étapes biochimiques de ce mécanisme s'assimilent plus à celles de l'apoptose. Ce mécanisme de mort cellulaire intervient par exemple dans l'ischémie.

C'est donc aussi un des buts de la présente invention que de disposer de nouveaux médicaments qui pourraient permettre de prévenir et/ou traiter la nécrose et/ou l'apoptose pathologique et/ou la nécroptose (médicaments antinécrotiques et/ou antiapoptotiques et/ou antinécroptotiques).

Les processus dégénératifs cellulaires peuvent résulter, entre autres, de situations pathologiques regroupées sous le terme de maladies ou affections dégénératives, de traumatismes, ou d'exposition à divers facteurs.

Ces traumatismes et facteurs peuvent inclure, par exemple, l'exposition aux radiations (UV, gamma), l'hypoxie

ou la privation d'oxygène, la privation de nutriments, la privation de facteurs de croissance, des poisons, des toxines cellulaires, des déchets, des toxines environnementales, des radicaux libres, des oxygènes réactifs ou encore certains événements et/ou procédures médicaux comme par exemple les traumatismes chirurgicaux incluant les transplantations de cellules, de tissus et d'organes. On peut citer également des agents chimiques ou biologiques utilisés comme agents thérapeutiques dans le contexte de traitements médicaux comme par exemple des agents cytostatiques ou des agents anti-inflammatoires.

L'invention n'a pas pour but de traiter les causes extracellulaires des pathologies ou des processus dégénératifs pouvant aboutir à la mort cellulaire, mais bien les conséquences au niveau cellulaire desdits processus pathologiques ou des dites pathologies et particulièrement de protéger la cellule contre lesdites conséquences.

Parmi les situations pathologiques caractérisées par un processus dégénératif les plus importantes, autres que les affections neurologiques ou neurodégénératives auxquelles la présente invention ne s'adresse pas, on trouve :

les maladies des os, des articulations, du tissu conjonctif et du cartilage, telles que l'ostéoporose, l'ostéomyélite, les arthrites dont par exemple l'ostéoarthrite, l'arthrite rhumatoïde et l'arthrite psoriatique, la nécrose avasculaire, la fibrodysplasie ossifiante progressive, le rachitisme, le syndrome de Cushing ;
les maladies musculaires telles que la dystrophie musculaire, comme par exemple la dystrophie musculaire de Duchenne, les dystrophies myotoniques, les myopathies et les myasthénies ;
les maladies de la peau, telles que les dermatites, l'eczéma, le psoriasis, le vieillissement, ou encore les altérations de la cicatrisation ;
les maladies cardiovasculaires telles que l'ischémie cardiaque et/ou vasculaire, l'infarctus du myocarde, la cardiopathie ischémique, l'insuffisance cardiaque chronique ou aigue, la dysrythmie cardiaque, la fibrillation auriculaire, la fibrillation ventriculaire, la tachycardie paroxystique, l'insuffisance cardiaque, l'anoxie, l'hypoxie, les effets secondaires dus à des thérapies avec des agents anticancéreux ; les maladies circulatoires telles que l'athérosclérose, les scléroses artérielles, et les maladies vasculaires périphériques, les accidents vasculaires cérébraux, les anévrismes ;
les maladies hématologiques et vasculaires telles que : l'anémie, l'amyloïdose vasculaire, les hémorragies, la drépanocytose, le syndrome de la fragmentation des globules rouges, la neutropénie, la leucopénie, l'aplasie médullaire, la pancytopénie, la thrombocytopénie, l'hémophilie ;
les maladies du poumon incluant la pneumonie, l'asthme ; les maladies chroniques obstructives des poumons comme par exemple les bronchites chroniques et l'emphysème ;
les maladies du tractus gastro-intestinal, telles que les ulcères ;
les maladies du foie comme par exemple les hépatites particulièrement les hépatites d'origine virale ou ayant pour agent causal d'autres agents infectieux, les hépatites autoimmunes, les hépatites fulminantes, certains désordres métaboliques héréditaires, la maladie de Wilson, les cirrhoses, la stéatose hépatique non alcoolique, les maladies du foie dues à des toxines ou des médicaments ;
les maladies du pancréas comme par exemple les pancréatites aigues ou chroniques ;
les maladies métaboliques telles que les diabètes mellitus et insipide, les thyroïdites ;
les maladies des reins telles que par exemple les désordres rénaux aigus ou la glomérulonéphrite ;
les infections virales et bactériennes telle que la septicémie ;
les intoxications sévères par des agents chimiques, des toxines ou des médicaments ;
les affections dégénératives associées au Syndrome Immuno Déficitaire Acquis (SIDA) ;
les désordres associés au vieillissement, tel que le syndrome du vieillissement accéléré ;
les maladies inflammatoires, telles que la maladie de Crohn, la polyarthrite rhumatoïde ;
les maladies auto-immunes telles que le lupus érythémateux ;
les désordres dentaires tels que ceux aboutissant à la dégradation des tissus comme par exemple les périodontites ;
les maladies ou désordres ophtalmiques incluant les rétinopathies diabétiques, le glaucome, les dégénérescences maculaires, la dégénérescence rétinienne, la rétinite pigmentaire, les trous ou déchirures rétiniennes, le décollement rétinien, l'ischémie rétinienne, les rétinopathies aigues associées à un traumatisme, les dégénérescences inflammatoires, les complications post chirurgicales, les rétinopathies médicamenteuses, la cataracte ;
les désordres des voies auditives, tels que l'otosclérose et la surdité induite par des antibiotiques ;
les maladies associées aux mitochondries (pathologies mitochondriales), telles que l'ataxie de Friedrich, la dystrophie musculaire congénitale avec anomalie mitochondriale structurelle, certaines myopathies (syndrome des MELAS, syndrome de MERFF, syndrome de Pearson), le syndrome de MIDD (diabètes mitochondriaux et surdité), le syndrome de Wolfram, la dystonie.

Dans l'art antérieur, le document Smith et coll. (1965) ("Steroid lysis of protoplasts and effects of stabilizers and steroid antagonists" APPLIED MICROBIOLOGY, 13(5),706-12 CODEN: APMBAY; ISSN: 0003-6919, 1965) décrit les résultats de l'étude de l'activité de l'acide 3,5-seco-4-nor-5-cholestanon-3-oique sur des protoplastes de la bactérie

*Sarcina lutea.* Cette étude est réalisée dans le but de comprendre le mécanisme de l'activité antibactérienne dudit composé. Ce document décrit donc l'activité antibactérienne de l'acide 3,5-seco-4-nor-5-cholestanon-3-oique, c'est-à-dire une activité du composé sur un agent pathogène.

Ce document ne décrit ni ne suggère une quelconque activité sur les cellules humaines.

Le document WO 2005/007192 A2 (FIBROGEN INC [US]; GUENZLER-PUKALL VOLKMAR [US]; KLAUS STEPHEN J [US];) 27 janvier 2005 décrit des agents cytoprotecteurs qui inhibent l'enzyme HIF hydroxylase et permettent de protéger des cellules exposées à un stress, par ex. dans le cadre d'une maladie/ou d'un accident cardiaque. Les composés décrits ne sont pas des dérivés du 3, 5-seco-4-norcholestane de formule (1).

Le document WO 2004/106370 A (THERAPTOSIS SA [FR]; ROBARTS RES INST [CA]; JACOTOT ETIENNE [FR]; MCFA) 9 décembre 2004 décrit des peptides cytoprotecteurs antiapoptotiques pouvant être utilisés dans le traitement de maladies telles que par ex. l'ischémie cardiaque. Les composés décrits ne sont pas des dérivés du 3, 5-seco-4-norcholestane de formule (1).

La demande WO2006/027454 divulgue l'utilisation de dérivés du 3,5-seco-4-norcholestane et leurs utilisations comme médicament, particulièrement comme médicament neuroprotecteur.

L'invention s'intéresse également à la protection des cellules, des tissus et/ou des organes transplantés, que ce soit avant, pendant (prélèvement, transport et/ou réimplantation) ou après la transplantation.

On recherche toujours des composés pharmacologiquement actifs pour lutter contre les processus dégénératifs évoqués ci-dessus.

La présente invention répond à cette demande de composés cytoprotecteurs. En effet, la demanderesse a découvert que les dérivés du 3,5-seco-4-norcholestane et notamment le 3,5-seco-4-nor-cholestan-5-one oxime-3-ol et l'un de ses esters sont doués de remarquables propriétés cytoprotectrices.

C'est pourquoi la présente invention a pour objet l'utilisation d'au moins un composé répondant à la formule I

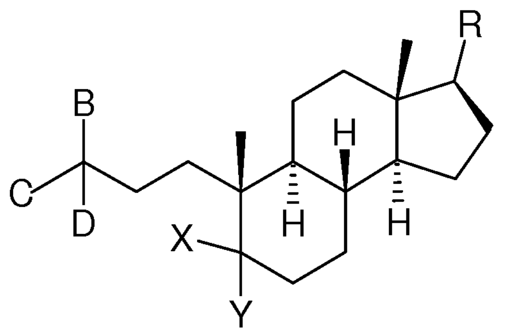

(I)

dans laquelle

- X et Y pris ensemble représentent une fonction céto (=O), un groupement oxime (=NOH) ou un groupement méthyloxime (=NHOMe) ou X représente un hydroxyle et Y un atome d'hydrogène,
- B représente un radical hydroxyle et C et D, identiques ou différents, représentent un atome d'hydrogène, ou un radical alkyle, linéaire ou ramifié, comprenant de 1 à 4 atomes de carbone,

ou B et C pris ensemble représentent une fonction céto et D un radical méthyle, hydroxyle, ou méthylamine,
ou B et C représentent un atome d'hydrogène et D un radical méthylamine,
ou B et C pris ensemble représentent un groupement oxime et D un radical méthyle,
et R représente un radical alkyle, linéaire ou ramifié, comprenant de 1 à 10 atomes de carbone,
ou l'un de ses sels d'addition avec les acides pharmaceutiquement acceptables, ou l'un de ses esters ou l'un des sels d'addition avec les acides pharmaceutiquement acceptables desdits esters,
pour la préparation d'un médicament cytoprotecteur contre les conséquences au niveau cellulaire de l'ostéoporose, l'ostéomyélite, les arthrites, l'ostéoarthrite, l'arthrite rhumatoïde et l'arthrite psoriatique, la nécrose avasculaire, la fibrodysplasie ossifiante progressive, le rachitisme, le syndrome de Cushing ; la dystrophie musculaire de Duchenne, les dystrophies myotoniques, les myopathies et les myasthénies ; les dermatites, l'eczéma, le psoriasis, l'ischémie cardiaque, l'infarctus du myocarde, la cardiopathie ischémique, l'insuffisance cardiaque chronique ou aigue, la dysrythmie cardiaque, la fibrillation auriculaire, la fibrillation ventriculaire, la tachycardie paroxystique, l'insuffisance cardiaque, l'anoxie, l'hypoxie, les effets secondaires dus à des thérapies avec des agents anticancéreux ; l'athérosclérose, les scléroses artérielles, les maladies vasculaires périphériques, les anévrismes ; l'anémie, l'amyloïdose vasculaire, les hémorragies, la drépanocytose, le syndrome de la fragmentation des globules rouges, la neutropénie, la leucopénie,

l'aplasie médullaire, la pancytopénie, la thrombocytopénie, l'hémophilie ; la pneumonie, l'asthme ; les maladies chroniques obstructives des poumons comme par exemple les bronchites chroniques et l'emphysème ; les ulcères du tractus gastro-intestinal ; les hépatites particulièrement les hépatites d'origine virale ou ayant pour agent causal d'autres agents infectieux, les hépatites autommunes, les hépatites fulminantes, la maladie de Wilson, les cirrhoses, la stéatose hépatique non alcoolique, les maladies du foie dues à des toxines ou des médicaments ; les pancréatites aigues ou chroniques ; les diabètes mellitus et insipide, les thyroïdites ; les désordres rénaux aigus ou la glomérulonéphrite ; la septicémie ; les intoxications sévères par des agents chimiques, des toxines ou des médicaments ; le syndrome du vieillissement accéléré ; la maladie de Crohn, la polyarthrite rhumatoïde ; le lupus érythémateux ; les périodontites ; l'otosclérose, la surdité induite par des antibiotiques ; la dystrophie musculaire congénitale avec anomalie mitochondriale structurelle, le syndrome des MELAS, le syndrome
de MERFF, le syndrome de Pearson, le syndrome de MIDD, le syndrome de Wolfram, la dystonie ;
à l'exception de la préparation d'un médicament neuroprotecteur.

Selon l'invention, les sels d'addition avec les acides pharmaceutiquement acceptables peuvent être par exemple des sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques, ou carboxyliques.

Selon l'invention le groupement oxime représente les isomères syn et anti purs ou en mélange associés à l'orientation de la liaison N-O par rapport à la double liaison C=N.

Selon une forme particulière de l'invention, le radical R des composés de formule I utilisables est préférentiellement le radical du cholestane de formule II

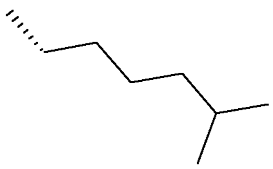

(II)

Selon d'autres formes particulières de l'invention, les composés de formule I pour lesquels X et Y pris ensemble représentent une fonction céto ou représentent un groupement oxime, sont préférentiellement utilisés.

Selon encore d'autres formes particulières de l'invention, les composés de formule I pour lesquels B représente un radical hydroxyle et C et D, identiques ou différents, représentent un atome d'hydrogène ou un radical alkyle, linéaire ou ramifié, comprenant de 1 à 4 atomes de carbone, ou encore pour lesquels B et C pris ensemble représentent une fonction céto et D représente un radical méthyle, sont préférentiellement utilisés.

Très préférentiellement, on utilise selon l'invention au moins un composé de formule I choisi parmi
le 3,5-seco-4-nor-cholestan-5-one oxime-3-ol,
le 3,5-seco-4-nor-cholestan-5-one oxime-3-méthyl alcool, ou
le 3,5-seco-4-nor-cholestan-5-one oxime-3-diméthyl alcool,
ou l'un de ses sels d'addition avec les acides pharmaceutiquement acceptables, ou l'un de ses esters ou l'un des sels d'addition avec les acides pharmaceutiquement acceptables desdits esters.

Les intéressantes propriétés cytoprotectrices des composés de formule I justifient leur utilisation pour la préparation d'un médicament cytoprotecteur, particulièrement destiné au traitement ou à la prévention de la nécrose et/ou de l'apoptose et/ou de la nécroptose (médicaments antinécrotiques et/ou antiapoptotiques et/ou antinécroptotiques) ou encore des affections comme
les maladies des os, des articulations, du tissu conjonctif et du cartilage,
les maladies musculaires,
les maladies de la peau,
les maladies cardiovasculaires,
les maladies circulatoires,
les maladies hématologiques et vasculaires,
les maladies du poumon,
les maladies du tractus gastro-intestinal,
les maladies du foie,
les maladies du pancréas,
les maladies métaboliques,
les maladies des reins,

les infections virales et bactériennes,
les intoxications sévères,
les affections dégénératives associées au Syndrome Immuno Déficitaire Acquis (SIDA),
les désordres associés au vieillissement,
les maladies inflammatoires,
les maladies auto-immunes,
les désordres dentaires,
les maladies ou désordres ophtalmiques,
les maladies des voies auditives,
les maladies associées aux mitochondries (pathologies mitochondriales).

L'invention s'intéresse également à la protection des cellules, des tissus ou des organes transplantés, que ce soit avant, pendant (prélèvement, transport et/ou réimplantation) ou après la transplantation.

Avantageusement, les composés de formule I peuvent être utilisés dans la préparation d'un médicament destiné à la protection des cellules cardiaques (médicament cardioprotecteur), à la protection des cellules hépatiques (médicament hépatoprotecteur) ou d'un médicament destiné au traitement ou à la prévention des maladies associées aux mitochondries.

Selon l'invention le composé de formule I est avantageusement présent dans le médicament cytoprotecteur à des doses physiologiquement efficaces ; lesdits médicaments renferment notamment une dose cytoprotectrice efficace d'au moins un des composés de formule I.

A titre de médicaments, les composés répondant à la formule I, leurs esters, leurs sels d'addition avec les acides pharmaceutiquement acceptables ainsi que les sels d'additions avec les acides pharmaceutiquement acceptables desdits esters peuvent être formulés pour la voie digestive ou parentérale.

Les médicaments selon l'invention peuvent comprendre en outre au moins un autre ingrédient thérapeutiquement actif, qu'il soit actif sur la même pathologie ou sur une pathologie différente, pour une utilisation simultanée, séparée ou étalée dans le temps, notamment lors d'un traitement chez un sujet atteint de l'une des pathologies précédemment citées.

Selon l'invention, le composé de formule I peut être utilisé dans le médicament, en mélange avec un ou plusieurs excipients ou véhicules inertes, c'est à dire pharmaceutiquement inactifs et non toxiques. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, etc. Des agents ou véhicules utilisables dans des formulations (liquides et/ou injectables et/ou solides) sont notamment la méthylcellulose, l'hydroxyméthylcellulose, la carboxyméthylcellulose, les cyclodextrines, le polysorbate 80, le mannitol, la gélatine, le lactose, des huiles végétales ou animales, l'acacia, etc. Les compositions peuvent être formulées sous forme de suspension injectable, de gels, huiles, comprimés, suppositoires, poudres, gélules, capsules, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.

L'administration peut être réalisée par toute méthode connue de l'homme du métier, de préférence par voie orale ou par injection, typiquement par voie intra-péritonéale, intra-cérébrale, intra-thécale, intra-veineuse, intra-artérielle ou intra-musculaire. L'administration par voie orale est préférée. S'agissant d'un traitement à long terme, la voie d'administration préférée sera sublinguale, orale ou transcutanée.

Pour les injections, les composés sont généralement conditionnés sous forme de suspensions liquides, qui peuvent être injectées au moyen de seringues ou de perfusions, par exemple. Il est entendu que le débit et/ou la dose injectée, ou de manière générale la dose à administrer, peuvent être adaptés par l'homme du métier en fonction du patient, de la pathologie, du mode d'administration, etc.. Il est entendu que des administrations répétées peuvent être réalisées, éventuellement en combinaison avec d'autres ingrédients actifs et/ou tout véhicule acceptable sur le plan pharmaceutique (tampons, solutions saline, isotonique, en présence d'agents stabilisants, etc.).

L'invention est utilisable chez les mammifères, notamment chez l'être humain.

En général la dose journalière du composé sera la dose minimale pour obtenir l'effet thérapeutique souhaité. Les doses des composés ci-dessus décrits et par exemple du 3,5-seco-4-nor-cholestan-5-one oxime- 3-ol seront en général comprises entre 0,001 à 100 mg par kilo par jour pour l'homme.

Si nécessaire, la dose journalière peut être administrée en deux, trois, quatre, cinq, six ou plus, prises par jour ou par sous-doses multiples administrées par intervalles appropriés pendant la journée.

La quantité choisie dépendra de multiples facteurs, en particulier de la voie d'administration, de la durée d'administration, du moment de l'administration, de la vitesse d'élimination du composé, du ou des différents produits utilisés en combinaison avec le composé, de l'âge, du poids et de la condition physique du patient, ainsi que de son histoire médicale, et de toutes autres informations connues en médecine.

La prescription du médecin traitant pourra commencer à des doses inférieures à celles généralement utilisées,

puis ces doses seront progressivement augmentées afin de mieux maîtriser l'apparition d'éventuels effets secondaires.

Les compositions selon l'invention, notamment les compositions pharmaceutiques ou médicaments, peuvent comprendre au moins un composé de formule I tels que décrits précédemment, ou l'un de ses sels d'addition avec les acides pharmaceutiquement acceptables, ou l'un de ses esters ou l'un des sels d'addition avec les acides pharmaceutiquement acceptables desdits esters,

Les compositions pharmaceutiques selon l'invention peuvent comprendre en outre au moins un autre ingrédient thérapeutiquement actif, pour une utilisation simultanée, séparée ou étalée dans le temps, notamment lors d'un traitement chez un sujet atteint de l'une des pathologies précédemment citées.

Les compositions pharmaceutiques selon l'invention peuvent avantageusement comprendre un ou plusieurs excipients ou véhicules inertes, c'est à dire pharmaceutiquement inactifs et non toxiques.

Les composés de formule I utilisés selon l'invention peuvent être synthétisés en faisant réagir un composé de formule III

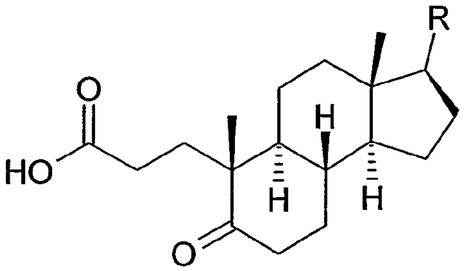

(III)

dans laquelle R a les significations précédemment indiquées que l'on soumet

- soit
  à l'action de la méthylamine puis de l'hydroxylamine pour obtenir un composé de formule I dans laquelle R à les significations précédemment indiquées, X et Y représentent ensemble un groupement oxime, B représente ensemble avec C une fonction céto et D un groupement méthyl amine
- soit à une méthylation pour obtenir un composé de formule IV

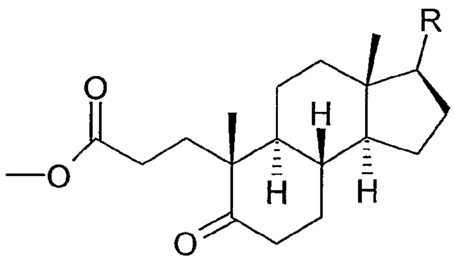

(IV)

dans laquelle R à les significations précédemment indiquées, que l'on soumet à l'action d'un agent de protection de la fonction cétone en position 5 pour obtenir un composé de formule V

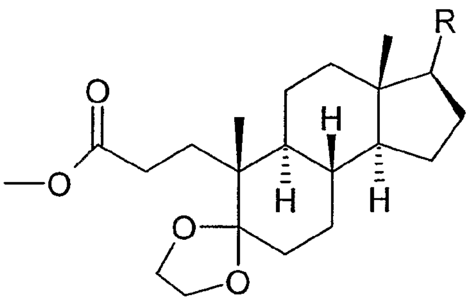

(V)

dans laquelle R à les significations précédemment indiquées, que

- ou bien
  l'on fait réagir avec le méthyl lithium puis l'on soumet à l'action d'un agent de déprotection de la fonction cétone en position 5 puis l'on fait réagir avec l'hydroxylamine pour obtenir un composé de formule I dans laquelle R a les significations précédemment indiquées, X et Y représentent ensemble un groupement oxime, B représente un groupement hydroxyle et C et D représentent des radicaux alkyles linéaires ou ramifiés de 1 à 4 atomes de carbone,
- ou bien
  l'on saponifie, puis fait réagir avec un composé de formule $H_3C$-NH-$OCH_3$, puis fait réagir avec le méthyl lithium, pour obtenir un composé de formule VI

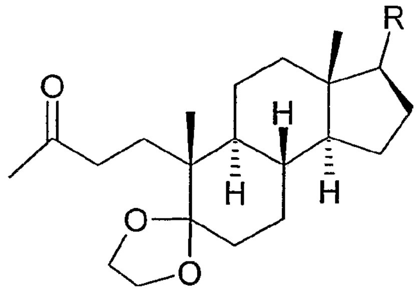

(VI)

que l'on soumet à une réduction de la fonction cétone puis l'on soumet à l'action d'un agent de déprotection de la fonction cétone en position 5 puis l'on fait réagir avec l'hydroxylamine pour obtenir un composé de formule I dans laquelle R a les significations précédemment indiquées, X et Y représentent ensemble un groupement oxime, B représente un groupement hydroxyle, et C représente un radical alkyle linéaire ou ramifié de 1 à 4 atomes de carbone éventuellement substitué et D représente un atome d'hydrogène,
- ou bien
  l'on réduit pour obtenir un composé de formule VII

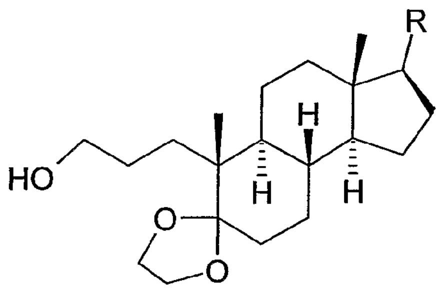

(VII)

dans laquelle R à les significations précédemment indiquées, B représente un groupement hydroxyle, et C et D représentent un atome d'hydrogène, que

- soit
  l'on soumet à l'action d'un agent d'oxydation pour obtenir un composé de formule VIII

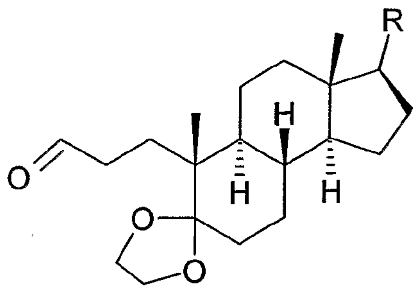

(VIII)

dans laquelle R a les significations précédemment indiquées, dont l'on prépare la base de Schiff puis réduit, puis

l'on soumet à l'action d'un agent de déprotection de la fonction cétone en position 5, puis l'on fait réagir avec l'hydroxylamine pour obtenir un composé de formule I dans laquelle R a les significations précédemment indiquées, X et Y représentent ensemble un groupement oxime, B représente un groupement méthylamine, et C et D représentent un atome d'hydrogène,

- soit
  l'on soumet à l'action d'un agent de déprotection de la fonction cétone en position 5, puis l'on fait réagir avec une amine choisie parmi l'hydroxylamine, la méthylhydroxylamine et la carboxyméthylhydroxylamine pour obtenir un composé de formule I dans laquelle R a les significations précédemment indiquées, X et Y représentent ensemble respectivement un groupement oxime, méthyloxime, et carboxyméthyloxime, B représente un groupement hydroxyle, et C et D représentent un atome d'hydrogène,
  et l'on isole et si désiré salifie les composés de formule I ou on les estérifie, les isole puis si désiré les salifie.

Dans des conditions préférentielles de mise en oeuvre du procédé ci-dessus décrit,

- la réaction du composé de formule III avec la méthylamine est réalisée en présence d'un agent de couplage activant la fonction acide comme le BOP (Benzotriazole-1-yl-oxy-tris-(diméthylamino)-phosphonium hexafluorophosphate) ou le TBTU (2-(1H-benzotriazole-1-yl)-1,1,3,3-tétraméthyluronium tétrafluoroborate) avantageusement en présence d'une base comme la N-méthylmorpholine, notamment dans un solvant adapté comme le dichlorométhane ou le diméthylformamide. De préférence on la réalise en présence de EDCI (1-Ethyl-3-(3'-diméthylaminopropyl)carbodiimide) associé à la 4-diméthylaminopyridine dans le dichlorométhane, le mélange étant soumis à une agitation à température ambiante pendant 24 h. Le produit est ensuite mis en solution de préférence dans la pyridine, puis on ajoute de 5 à 7 et notamment 6 équivalents de chlorhydrate d'hydroxylamine.
- la méthylation du composé de formule III est réalisée par réaction avec le méthanol en présence de chlorure de thionyle, de préférence en solubilisant l'acide de formule II dans un volume adapté d'un mélange de 70% de méthanol et 30% de dichlorométhane. On refroidit à 0°C et l'on ajoute goutte-à-goutte 3 équivalents de chlorure de thionyle. On agite alors 2 heures à température ambiante. Sur ce composé, la protection de la fonction cétone est effectuée de préférence en solubilisant le produit dans un excès, par exemple 10 équivalents de triméthylorthoformate et un volume suffisant d'éthylène glycol, puis addition d'acide p-toluènesulfonique anhydre.
- la réaction du composé de formule V avec le méthyl lithium est de préférence effectuée dans le THF anhydre puis après refroidissement à environ - 45 °C, addition goutte-à-goutte d'un excès de méthyl lithium. La déprotection du dioxolane qui bloque la fonction cétone en position 5, est réalisée dans l'acétone en présence d'acide sulfurique. De préférence on l'effectue dans du dioxane, en présence d'un mélange eau /acide acétique 1/1. L'oxime de la cétone est avantageusement réalisée comme ci-dessus.
- la saponification du composé de formule V est réalisée avec la soude de préférence dans le dioxane. On ajoute notamment environ 2 équivalents d'une solution aqueuse de soude. Ce produit est mis en réaction avec un composé de formule $H_3C\text{-}NH\text{-}OCH_3$ par exemple en présence d'un agent de couplage activant la fonction acide comme le BOP ou le TBTU en présence d'une base comme la N-méthylmorpholine dans un solvant adapté comme le dichlorométhane ou le diméthylformamide. De préférence on la réalise en présence de EDCI associé à l'hydroxybenzotriazole avec de la triéthylamine ajoutée goutte-à-goutte dans le solvant. Ce produit est mis en réaction avec le méthyl lithium sous atmosphère d'argon selon le protocole décrit ci-dessus, puis la fonction cétone en 3 est réduite par le borohydrure de sodium. Le produit obtenu est ensuite soumis à la déprotection de la fonction cétone en position 5 et mis en réaction avec l'hydroxylamine selon le même protocole décrit ci-dessus.
- la réduction du composé de formule V pour obtenir le composé de formule VII est réalisée de préférence par de l'hydrure d'aluminium lithium notamment en le plaçant en suspension dans du tétrahydrofurane. On hydrolyse avec précaution par addition d'une solution de sulfate de sodium.
- l'oxydation du composé de formule VII est réalisée à l'aide de chlorochromate de pyridimium. Sur ce produit on obtient la base de Schiff qui est réduite instantanément notamment en solubilisant sous argon de préférence dans l'éthanol en présence de triéthylamine, de chlorhydrate de méthylamine et de tétraisopropoxyde de titane, puis addition de borohydrure de sodium. La déprotection de la fonction cétone en position 5 ainsi que la réaction avec l'hydroxylamine sont effectuées dans les conditions précédemment décrites.

Les exemples qui suivent illustrent la présente demande sans toutefois la limiter.

Exemples

Les temps de rétention ci-après sont exprimés en minutes et centièmes de minute.
La méthode de chromatographie liquide utilisée pour l'ensemble des produits est la suivante :

Colonne : Macherey-Nagel - Nucleosil® 300-6 C4 - 150x4,6 mm Gradient : eau (+0.05% acide trifluoroacétique) / acétonitrile (+0,05% acide trifluoroacétique)

t = 0 min : 60% acétonitrile, 40% $H_2O$
t = 6 min : 100% acétonitrile, 0% $H_2O$
t = 11 min : 100% acétonitrile, 0% $H_2O$
t = 13 min : 60% acétonitrile, 40% $H_2O$
t = 15 min : 60% acétonitrile, 40% $H_2O$.

Les conditions d'ionisation pour le spectromètre de masse sont :

Température de la source : 250°C
Tension de cône : 50 V
Capillaire voltage : 3 kV
Rf lens : 0.3 V

**Exemple 1 : synthèse de la 3,5-seco-4-nor-cholestane-5-one, oxime-3-methylamide**

Etape A : Dans un premier temps, dans un ballon on introduit 250 mg de 3,5-seco-4-nor-cholestane-5-one, oxime-3-oic acide, 38 mg de chlorhydrate de méthylamine, 250 mg d'EDCI, 100 mg de DMAP et 2,5 mL de dichlorométhane. La solution est agitée 24 heures à température ambiante puis le milieu réactionnel est dilué par ajout de dichlorométhane et lavé avec une solution de bicarbonate de sodium à 10%. La phase organique est séchée sur sulfate de magnésium puis concentrée sous pression réduite. Le résidu obtenu est purifié par flash chromatographie ($CH_2Cl_2$ / MeOH 95/5). On récupère 176 mg de 3,5-seco-4-nor-cholestane-5-one -3-méthylamide avec un rendement de 68%.
Analyse

RMN-1 H (CDCl3) : conforme
Temps de rétention : 4 min 42 centièmes
Pics détectés en spectrométrie de masse : [M+H]+ = 418 ; [2M+H]+=835

Etape B : Ensuite, dans un ballon, on introduit 50 mg de 3,5-seco-4-nor-cholestane-5-one-3-méthylamide, 50 mg de chlorhydrate d'hydroxylamine dans 1 mL de pyridine. On agite 16 heures à température ambiante puis le milieu réactionnel est concentré sous pression réduite. Le résidu obtenu est repris dans un mélange $CH_2Cl_2$ / $H_2O$ ; la phase organique est séparée, lavée à l'eau, séchée sur sulfate de sodium anhydre et concentrée sous pression réduite. On récupère 40,6 mg de 3,5-seco-4-nor-cholestane-5-one, oxime-3-méthylamide avec un rendement de 78%.
Analyse

RMN-1 H ($CDCl_3$) : conforme
Temps de rétention : 3 min 70 centièmes
Pics détectés en spectrométrie de masse : [M+H]+= 433 ; [2M+H]+=865

**Exemple 2 : synthèse du 3,5-seco-4-nor-cholestane-5-one-3-dimethyl alcool**

Etape A : Dans un ballon, on solubilise 10,5 g d'acide 3,5-seco-4-nor-cholestane-5-one-3-oique dans 378 mL de méthanol et 146 mL de dichlorométhane. On refroidit à 0°C et l'on ajoute goutte-à-goutte 5,7 mL de chlorure de thionyle. On agite alors 2 heures à température ambiante. Le milieu réactionnel est concentré sous pression réduite, co-évaporé au toluène puis au dichlorométhane. On obtient 10,3 g de 3,5-seco-4-nor-cholestane-5-one-3-méthyl ester avec rendement de 94%. Le produit est utilisé tel quel sans purification.

RMN-1H ($CDCl_3$) : conforme

Temps de rétention : 4 min 69 centièmes
Pic détecté en spectrométrie de masse : {M+H}+ = 419 ; [2M+H]+ = 785

Etape B : Dans un ballon, on met en solution 9,62 g de 3,5-seco-4-nor-cholestane-5-one-3-méthyl ester dans 25 mL de triméthylorthoformate et 53 mL d'éthylène glycol. On ajoute alors 400 mg (2,3 mmol) d'acide p-toluènesulfonique anhydre puis on agite 1 nuit à température ambiante. On ajoute de l'acétate d'éthyle au milieu réactionnel ; on réalise un lavage par une solution à 10% d'hydrogénocarbonate de sodium. La phase organique est séparée, séchée sur sulfate de magnésium anhydre et concentrée sous pression réduite. On obtient 9,95 g de 3,5-seco-4-nor-cholestane-5,5(éthylène dioxy)-3-méthyl ester avec rendement de 93%. Le produit est utilisé tel quel sans purification.

RMN-1H ($CDCl_3$) : conforme
Temps de rétention : 5 min 76 centièmes
Pic détecté en spectrométrie de masse : {M+H}+ = 463

Etape C : Dans un ballon, on solubilise 300 mg de 3,5-seco-4-nor-cholestane-5,5(éthylène dioxy)-3-méthyl ester dans 5 mL de THF anhydre. Le milieu est refroidi à -45 °C puis on ajoute goutte-à-goutte 1,36 mL d'une solution de méthyllithium 1,6M dans l'éther. Après 30 minutes d'agitation à -45°C. On ajoute quelques gouttes de méthanol au milieu réactionnel et ramène à température ambiante. On reprend dans 20 mL d'éther diéthylique et on lave avec une solution saturée de bicarbonate de sodium, puis avec une solution saturée de chlorure de sodium. La phase organique est séchée sur sulfate de magnésium, puis concentrée sous pression réduite. On obtient 295 mg 3,5-seco-4-nor-cholestane-5,5(éthylène dioxy)-3-diméthyl alcool (PM=462) avec un rendement de 98%.

Temps de rétention : 5 min 56 centièmes
Pics détectés en spectrométrie de masse : [M- ($CH_2OH$-$CH_2OH$ + $H_2O$)+H]+]=401

Etape D : Dans un ballon, on ajoute 6 mL d'un mélange eau /acide acétique 1/1 et 295 mg de 3,5-seco-4-nor-cholestane-5,5(éthylène dioxy)-3-diméthyl alcool ; on chauffe à reflux pendant 1h30. Après refroidissement, le milieu réactionnel est dilué avec de l'acétate d'éthyle, lavé avec une solution de saturée de chlorure de sodium, puis avec une solution saturée de bicarbonate de sodium. Enfin, la phase organique est séchée sur sulfate de magnésium et concentrée sous pression réduite. Le produit brut obtenu est purifié par flash chromatographie (éther de pétrole / acétate d'éthyle 8/2). On obtient 180 mg 3,5-seco-4-nor-cholestane-5-one-3-diméthyl alcool avec un rendement de 68%.

RMN-1H ($CDCl_3$) : conforme
Temps de rétention : 5 min 08 centièmes
Pics détectés en spectrométrie de masse: [M+H]+= 419 ; [M-H2O+H]+=401 ; [2M+H]+=837

**Exemple 3: synthèse du 3,5-seco-4-nor-cholestane-5-one, oxime-3-diméthyl alcool**

Dans un ballon, on introduit 1 g du composé de l'exemple 2, 1 g de chlorhydrate d'hydroxylamine dans 53 mL de pyridine et quelques mL de dichlorométhane pour solubiliser la cétone. On agite 16 heures à température ambiante puis le milieu réactionnel est concentré sous pression réduite. Le résidu obtenu est repris dans un mélange $CH_2Cl_2$ / $H_2O$ ; la phase organique est séparée, lavée à l'eau, séchée sur sulfate de sodium anhydre et concentrée sous pression réduite. On récupère 814 mg de 3,5-seco-4-nor-cholestane-5-one, oxime-3-diméthyl alcool avec un rendement de 78%.

RMN-1 H ($CDCl_3$) : conforme
Temps de rétention : 5 min 09 centièmes
Pics détectés en spectrométrie de masse : [M+H]+= 434 ; [2M+H]+=867

**Exemple 4: synthèse du 3,5-seco-4-nor-cholestane-5-one, oxime- 3-méthyl alcool**

Etape A : Dans un ballon, on place 2 g de 3,5-seco-4-nor-cholestane-5,5(éthylène dioxy)-3-méthyl ester dans 26 mL de dioxane. On ajoute 8,6 mL d'une solution de soude 1 N. Le milieu réactionnel est chauffé à reflux pendant 1h30 et le dioxane est évaporé sous pression réduite. On acidifie la solution obtenue par ajout d'une solution d'acide chlorhydrique 1 N jusqu'à pH=1 et l'on extrait 2 fois au toluène. Les phases organiques sont réunies, séchées sur

sulfate de magnésium anhydre et concentrées sous pression réduite. On récupère 1,92 g d'acide 3,5-seco-4-nor-cholestane-5,5(éthylène dioxy)-3-oïque avec un rendement de 99% qui est utilisé sans traitement supplémentaire dans l'étape suivante.

Etape B : Dans un ballon, on place 1,9 g d'acide 3,5-seco-4-nor-cholestane-5,5(éthylène dioxy)-3-oïque dans 30 mL de dichlorométhane. On ajoute à cette solution 1,06 g d'EDCI, 743 mg d'HOBT, 537 mg de chlorhydrate de N,O-diméthylhydroxylamine puis 1.37 mL de triéthylamine goutte-à-goutte. On agite à température ambiante pendant 16 heures. On ajoute un mélange $CH_2Cl_2/H_2O$ au milieu réactionnel et l'on extrait 3 fois au dichlorométhane. Les phases organiques sont réunies, séchées sur sulfate de magnésium anhydre et concentrées sous pression réduite. Le résidu obtenu est purifié par flash chromatographie ($CH_2Cl_2$/acétate d'éthyle 8/2). On récupère 1,46 g 3,5-seco-4-nor-cholestane-5,5(éthylène dioxy)-3-(N,N-méthoxy-méthyl) amide avec un rendement de 70%.

RMN-1 H ($CDCl_3$) : conforme
Temps de rétention : 5 min 31 centièmes
Pic détecté en spectrométrie de masse : [M+H]+= 492

Etape C : dans un ballon sous argon, on introduit 1,4 g de 3,5-seco-4-nor-cholestane-5,5(éthylène dioxy)-3-(N,N-méthoxy-méthyl) amide dans 20 mL de tétrahydrofurane anhydre et l'on refroidit à 0°C. On ajoute alors goutte-à-goutte 3,38 mL d'une solution de méthyllithium 1.6M dans l'éther. Le milieu réactionnel est agité 3h40 à 0°C puis on ajoute goutte-à-goutte une solution de 0.72 mL d'acide chlorhydrique concentré dans 7.28 mL d'eau. Le tétrahydrofurane est évaporé sous pression réduite ; la solution aqueuse obtenue est basifiée par ajout de soude 1 N jusqu'à pH=10. On extrait à l'éther diéthylique ; les phases organiques sont réunies, séchées sur sulfate de magnésium anhydre, et concentrées sous pression réduite. Le résidu obtenu est purifié par flash chromatographie (éther de pétrole/acétate d'éthyle 9/1). On récupère 930 mg de 3,5-seco-4-nor-cholestane-5,5(éthylène dioxy)-3-méthyl cétone avec un rendement de 73%.

RMN-1 H ($CDCl_3$) : conforme
Temps de rétention : 5 min 65 centièmes
Pic détecté en spectrométrie de masse : [M+H]+= 403

Etape D : Dans un ballon, on place 119 mg de 3,5-seco-4-nor-cholestane-5,5(éthylène dioxy)-3-méthylcétone de l'étape C dans 1,5 mL de méthanol. On refroidit à 0°C et l'on ajoute 10 mg de borohydrure de sodium. Le milieu réactionnel est agité à 0°C pendant 1h puis concentré sous pression réduite. Le résidu est repris dans de l'eau et extrait au dichlorométhane. La phase organique est séchée sur sulfate de magnésium et concentrée sous pression réduite. On récupère 94 mg 3,5-seco-4-nor-cholestane-5,5(éthylène dioxy)-3-méthylalcool avec un rendement de 78%, ce produit est utilisé tel quel.

RMN-1 H ($CDCl_3$) : conforme
Temps de rétention : 5 min 22 centièmes
Pics détectés en spectrométrie de masse : [M+H]+= 387

Etape E : On opère comme à l'étape D de l'exemple 2 pour déprotéger la cétone en 5.

Etape F : Dans un ballon on introduit 121 mg de 3,5-seco-4-nor-cholestane-5-one-3-méthyl alcool, 1,5 mL de pyridine et 121 mg de chlorhydrate d'hydroxylamine. La solution est agitée deux jours à température ambiante. Le milieu réactionnel est concentré sous pression réduite, repris dans l'eau et extrait au dichlorométhane. La phase organique est ensuite lavée à l'eau, puis séchée sur sulfate de magnésium et concentrée sous pression réduite. Le produit ainsi obtenu est purifié par flash chromatographie (éther de pétrole/acétate d'éthyle 9/1). On obtient 66 mg de 3,5-seco-4-nor-cholestane-5-one, oxime-3-méthyl alcool avec un rendement de 53%.

RMN-1 H ($CDCl_3$) : conforme
Temps de rétention : 4 min 91 centièmes
Pics détectés en spectrométrie de masse : [M+H]+= 420

**Exemple 5 : synthèse de la 3,5-seco-4-nor-cholestane-5-one, oxime-3-méthylamine**

Etape A : Dans un ballon, on place en suspension 615 mg de LiAIH4 dans 57 mL de THF. On refroidit à 0°C et l'on ajoute goutte-à-goutte une solution de 3,0 g de 3,5-seco-4-nor-cholestane-5,5(éthylène dioxy)-3-méthyl ester, dans

57 mL de tétrahydrofurane. On agite alors à 0°C pendant 5h. On hydrolyse avec précaution par ajout d'une solution de sulfate de sodium ; la solution blanche obtenue est agitée 30 minutes, puis filtrée. Le filtrat est concentré sous pression réduite et repris dans de l'eau, extrait avec de l'acétate d'éthyle. La phase organique est séchée sur sulfate de magnésium puis concentrée sous pression réduite. On obtient 2,55 g 3,5-seco-4-nor-cholestane-5,5(éthylène dioxy)-3-ol avec un rendement de 85%, ce produit est utilisé tel quel.

RMN-1H ($CDCl_3$) : conforme
Temps de rétention : 4 min 82 centièmes
Pics détectés en spectrométrie de masse : [M- (CH2OH-CH2OH + H2O)+H]+= 373

Etape B : Dans un ballon sous argon, on solubilise 476 mg de 3,5-seco-4-nor-cholestane-5,5(éthylène dioxy)-3-ol, dans 7 mL de dichlorométhane, puis on ajoute 189 mg d'alumine neutre et 399 mg de chlorochromate de pyridinium ; on agite à température ambiante pendant 3h30. Le milieu réactionnel est filtré sur Célite® ; le filtrat est concentré sous pression réduite. Le résidu obtenu est purifié par flash chromatographie (toluène / acétate d'éthyle 9/1 puis 8/2). On obtient 328 mg de 3,5-seco-4-nor-cholestane-5,5(éthylène dioxy)-3-al avec un rendement de 69%.

Temps de rétention : 5 min 57 centièmes
Pics détectés en spectrométrie de masse : [M+H]+= 433

Etape C : Dans un ballon sous argon, on solubilise 323 mg de 3,5-seco-4-nor-cholestane-5,5(éthylène dioxy)-3-al dans 3 mL d'éthanol, puis on ajoute 209 μL de triéthylamine, 100 mg de chlorhydrate de méthylamine et 444 μL de tétraisopropoxyde de titane. Le milieu réactionnel est agité 6h à température ambiante, puis on ajoute 42,5 mg de borohydrure de sodium. On agite 16 heures à température ambiante. Le milieu réactionnel est filtré et lavé avec du dichlorométhane. Le filtrat est séché sur sulfate de magnésium et concentré sous pression réduite. Le résidu obtenu est purifié par flash chromatographie (dichlorométhane / méthanol 9/1 à 5/5). On obtient 84 mg de 3,5-seco-4-nor-cholestane-5,5(éthylène dioxy)-oxime-3-méthylamine avec un rendement de 25%.

RMN-1 H ($CDCl_3$) : conforme
Temps de rétention : 3 min 93 centièmes
Pics détectés en spectrométrie de masse : [M+H]+= 448

Etape D : Dans un ballon, on introduit 50 mg de 3,5-seco-4-nor-cholestane-5,5(éthylène dioxy)-3-méthylamine et 976 μL d'un mélange eau/acide acétique 1/1. Le mélange est porté au reflux pendant 6h. Après refroidissement, le milieu réactionnel est dilué avec de l'acétate d'éthyle et lavé avec une solution saturée de chlorure de sodium puis avec une solution de bicarbonate de sodium à 5%. La phase organique est séchée sur sulfate de magnésium et concentrée sous pression réduite. Le produit obtenu est purifié par flash chromatographie (dichlorométhane / méthanol 95/5) ; on obtient 5 mg 3,5-seco-4-nor-cholestane-5-one-3-méthylamine avec un rendement de 11%.

RMN-1 H ($CDCl_3$) : conforme
Temps de rétention : 3 min 68 centièmes
Pics détectés en spectrométrie de masse : [M+H]+= 404

Etape E : Dans un ballon, on introduit 5 mg de 3,5-seco-4-nor-cholestane-5-one-3-méthylamine, 5 mg de chlorhydrate d'hydroxylamine et 287 μL de pyridine. Le mélange est agité 16 heures à température ambiante. On reprend ensuite dans du dichlorométhane et on lave à l'eau. La phase organique est séchée sur sulfate de magnésium et concentrée sous pression réduite. On obtient 5 mg de 3,5-seco-4-nor-cholestane-5-one, oxime-3-méthylamine avec un rendement de 91%.

Temps de rétention : 3 min 66 centièmes
Pics détectés en spectrométrie de masse : [M+H]+ = 419

**Exemple 6 : synthèse du 3,5-seco-4-nor-cholestane-5-one, méthyloxime-3-ol**

Dans un ballon, on introduit 20 mg de 3,5-seco-4-nor-cholestane-5-one-3-ol, 20 mg de chlorhydrate de O-méthylhydroxylamine dans 1 mL de pyridine. On agite 36h à température ambiante et l'on rajoute 10 mg de chlorhydrate de O-méthylhydroxylamine. On agite à nouveau 16 heures à température ambiante puis le milieu réactionnel est concentré sous pression réduite. Le résidu obtenu est repris dans un mélange $CH_2Cl_2$ / $H_2O$ ; la phase organique est séparée, lavée à l'eau, séchée sur sulfate de magnésium anhydre et concentrée sous pression réduite. On obtient 18

mg d'une huile jaune qui est purifiée par flash chromatographie (éther de pétrole / acétate d'éthyle 9/1). On récupère 5,8 mg de 3,5-seco-4-nor-cholestane-5-one, methyloxime-3-ol avec un rendement de 27%.

Analyse

RMN-1H ($CDCl_3$) : conforme
Temps de rétention : 5 min 50 centièmes
Pic détecté en spectrométrie de masse : [M+H]+= 420

**Exemple 7 : synthèse du 3,5-seco-4-nor-cholestane-5-one carboxyméthyloxime-3-ol**

Dans un ballon, on introduit 52 mg de cétone, 25 mg d'hémi-chlorhydrate de carboxyméthoxylamine dans 0,5 mL de pyridine. On agite 2 jours à température ambiante puis le milieu réactionnel est concentré sous pression réduite. Le résidu obtenu est repris dans un mélange $CH_2Cl_2$ / $H_2O$ ; la phase organique est séparée, lavée à l'eau puis par une solution d'acide chlorhydrique à 2%, séchée sur sulfate de sodium anhydre et concentrée sous pression réduite. Le résidu obtenu est purifié par flash chromatographie (éther de pétrole / acétate d'éthyle 8/2). On obtient 24 mg avec un rendement de 39% de la carboxyméthyloxime.

Analyse

RMN-1H ($CDCl_3$) : conforme
Temps de rétention : 4 min 40 centièmes
Pic détecté en spectrométrie de masse : [M+H]+= 464 ; $[2M+H]^+$=927

**Exemple 8**

On a préparé une suspension répondant à la formule
3,5-seco-4-nor-cholestan-5-one oxime- 3-ol 20 mg par ml
Excipient : Emulsion huileuse

**Exemple 9**

On a préparé une forme sèche répondant à la formule
Chlorydrate de 3,5-seco-4-nor-cholestan-5-one
oxime- 3- N,N-diméthylglycine ester 250 mg
Excipient : qsp une gélule terminée à 750 mg

**Exemple 10 : synthèse du 3,5-seco-4-nor-cholestan-5-one oxime-3-N,N-diméthylglycine ester : prodrogue du 3,5-seco-4-nor-cholestan-5-one oxime-3-ol**

Dans un ballon, on place 509 mg de 3,5-seco-4-nor-cholestan-5-one-3-ol, 182 mg de chlorhydrate de N,N-diméthylglycine, 275 mg d'EDCI et 207 mg de DMAP dans 10 à 15 mL de dichlorométhane. On agite à température ambiante pendant 16 heures. On ajoute au milieu réactionnel une solution de bicarbonate de sodium à 5% et l'on extrait au dichlorométhane. Les phases organiques sont réunies, séchées sur sulfate de sodium anhydre et concentrées sous pression réduite. Le résidu obtenu est purifié par flash chromatographie (toluène / acétate d'éthyle 8/2). On récupère 488 mg avec un rendement de 78%.

Analyse

RMN-1 H ($CDCl_3$) : conforme
Temps de rétention : 3 min 77 centièmes
Pic détecté en spectrométrie de masse : [M+H]+= 476
Le produit est ensuite engagé dans la réaction suivante :

Dans un ballon, on introduit 488 mg du produit obtenu et 488 mg de chlorhydrate d'hydroxylamine dans 23 mL de pyridine. On agite 16 heures à température ambiante puis le milieu réactionnel est repris dans un mélange $CH_2Cl_2$ / $H_2O$ ; la phase organique est séparée, lavée à l'eau, séchée sur sulfate de sodium anhydre et concentrée sous pression réduite. On récupère 378 mg de l'oxime avec un rendement de 75%. Le produit est ensuite salifié en présence d'une solution d'éther acidifiée par une solution d'HCl, pour obtenir le produit sous forme de chlorhydrate.

Analyse

RMN-1 H ($CDCl_3$) : conforme
Temps de rétention : 3 min 43 centièmes
Pic détecté en spectrométrie de masse : [M+H]+= 491

**Exemple 11 : synthèse du 3,5-seco-4-nor-cholestan-5-one,oxime-3-(4-methyl-1-piperazine) propanoate ester : Prodrogue du 3,5-seco-4-nor-cholestan-5-one, oxime-3-ol**

Dans un ballon, on place 264 mg de 3,5-seco-4-nor-cholestan-5-one-3-ol, 121 mg d'acide 4-méthyl-1-piperazine propanoïque sous forme de sel de lithium, 1425 mg d'EDCI et 106 mg de DMAP dans 2 à 3 mL de dichlorométhane. On agite à température ambiante pendant 1 nuit. On ajoute de l'eau au milieu réactionnel et l'on extrait au dichlorométhane. Les phases organiques sont réunies, séchées sur sulfate de sodium anhydre et concentrées sous pression réduite. Le résidu obtenu est purifié par flash chromatographie (toluène / acétate d'éthyle 98/2). On récupère 54 mg du produit désiré avec un rendement de 15%.
Analyse

RMN-1 H ($CDCl_3$) : conforme
Temps de rétention : 3 min 66 centièmes
Pic détecté en spectrométrie de masse : [M+H]+= 545
Le produit est ensuite engagé dans la réaction suivante :

Dans un ballon, on introduit 30 mg du produit obtenu et 30 mg de chlorhydrate d'hydroxylamine dans 1.2 mL de pyridine. On agite 5h30 à température ambiante puis le milieu réactionnel est repris dans un mélange $CH_2Cl_2$ / $H_2O$ ; la phase organique est séparée, lavée à l'eau, séchée sur sulfate de sodium anhydre et concentrée sous pression réduite. On récupère 19 mg de l'oxime avec rendement de 13%.

Analyse

RMN-1 H ($CDCl_3$) : conforme
Temps de rétention : 3 min 62 centièmes
Pic détecté en spectrométrie de masse : [M+H]+= 560

Le produit est ensuite salifié en présence d'une solution d'éther acidifiée par une solution aqueuse d'acide chlorhydrique, pour obtenir le produit sous forme de di-chlorhydrate.

**Exemple 12 : Effet anti-apoptotique du 3,5-seco-4-nor-cholestan-5-one oxime-3-ol : Contractilité et apoptose des cardiomyocytes ventriculaires de lapin**

Les propriétés antiapoptotiques du 3,5-seco-4-nor-cholestan-5-one oxime-3-ol (Azasteroidal alkaloids. Synthesis of A-nor-B-homo-5-azacholestane. Rodewald, W. J.; Wicha, J. Univ. Warsaw, Bulletin de l'Académie Polonaise des Sciences, Série des Sciences Chimiques (1963), 11(8), 437-441) ont été analysées sur des cardiomyocytes, par un test de dysfonctionnement contractile induit par la doxorubicine.

➢ **Matériels et Méthodes**

**Composé à tester**

Une solution stock de 3,5-seco-4-nor-cholestan-5-one oxime-3-ol à la concentration de 10mM dans 100% de DMSO a été utilisée.
La concentration finale en DMSO a été la même pour tous les points expérimentaux, indépendamment des concentrations en molécules utilisées.
Le 3,5-seco-4-nor-cholestan-5-one oxime-3-ol a été testé aux concentrations de 0,1 et 0,3 $\mu$M, dilué dans une solution de Tyrode (composition en mmol/L: NaCl 135, KCL 5,4, $NaH_2PO_4$ 0,33, $CaCl_2$ 1,2, $MgCl_2$ 1,0, Hepes 10 ; pH ajusté à 7,4 avec NaOH).

**Obtention de cellules isolées de cardiomyocytes ventriculaires de lapin**

Des cellules ventriculaires isolées sont obtenues à partir de coeurs de lapins males de Nouvelle Zélande comme décrit dans A. d'Anglemont de Tassigny et al., Fund Clin Pharmacol, 18: 531-38, 2004. En bref, les lapins (2,0-2,5 kg) sont anesthésiés avec une solution de pentobarbital (50 mg/kg) puis reçoivent de l'héparine (200 IU/kg). Les coeurs sont excisés et immédiatement perfusés, pendant 10 à 15 minutes grâce à un appareil de Langendorff sans recirculation avec une solution isotonique de tyrode (sans calcium) oxygénée (95%, 2-5% $CO_2$) (en mM : NaCl 135, KCl 5,4, $Na_2PO_4$ 0,33, $MgCl_2$ 1,0, HEPES 10, pH ajusté à 7,4 avec NaOH 1 N à 37°C, 280-300 mOsmol/kg$H_2O$). Ensuite, tous les coeurs sont perfusés pendant 3 minutes en mode "recirculation" avec la même solution de Tyrode sans calcium (débit coronaire, 10-15 mL/min) additionnée de 1 mg/mL de collagénase type II et 0,28 mg/mL de protéase type XIV. Finalement, tous les coeurs sont perfusés en mode sans recirculation avec la même solution de Tyrode supplémentée avec 0,3 mM CaCl2 pendant 10 min. Le ventricule gauche est enlevé et découpé en petits morceaux, la dissociation cellulaire est réalisée par agitation mécanique douce. Du calcium extracellulaire est ajouté par incrément toutes les 15 minutes, pour arriver à une concentration physiologique de 1,0 mM. Les myocytes isolés sont maintenus dans un milieu sans sérum contenant (en mM) NaCl 110, KCl 5,4, $Na_2PO_4$ 0,33, $NaHCO_3$ 25, Glucose 5, $MgCl_2$ 0,8, $CaCl_2$ 1, pH ajusté à 7,4 jusqu'à 1h30 avant l'expérimentation. Toutes les cellules sont en forme de baguette, ont une striation croisée claire et ne présentent pas de vésicule à leur surface au microscope optique.

**Marquage à l'annexine V**

Le marquage à l'annexine V de la phosphatidylsérine a été utilisé comme méthode quantitative de mesure de l'apoptose en utilisant le kit MiniMacs cell isolation (Miltenyi Biotec, Bergisch, Gladbach, Germany). En bref, les cellules exposant de la phosphatidylsérine sont marquées magnétiquement avec des microbilles d'annexine V, puis passées dans une colonne placée dans un champ magnétique. Les cellules marquées (qui présentent de la phosphatidylsérine marquée magnétiquement) sont retenues dans la colonne alors que celles non marquées (cellules nécrotiques et non-apoptotiques) ne sont pas retenues. La colonne est retirée du champ magnétique, les cellules exposant de la phosphatidylsérine retenues magnétiquement sont éluées comme fraction positive et comptées avec une cellule de Mallassez. Le pourcentage de cellules apoptotiques est alors rapporté au nombre initial de cellules.

**Mesure de l'activité caspase-3**

L'activité caspase-3 est utilisée comme méthode quantitative de mesure de l'apoptose. En bref, les cellules sont lysées et le surnageant est utilisé pour la mesure d'activité caspase-3 en utilisant le kit AK-005 (Biomol Research Laboratories, Plymouth Meeting, PA, USA). Le substrat fluorogène pour mesurer l'activité caspase-3 (DEVD) est marqué avec le fluorochrome 7-amino-4-methyl coumarine (AMC) qui produit une fluorescence jaune-verte détectable à la lumière UV à 360/460 nm pendant 210 min. L'AMC est libéré du substrat par clivage par la caspase-3, l'expression de l'enzyme est exprimée en fmol/min.

**Mesure de la contractilité**

Les myocytes sont transférés dans une chambre à 37°C perfusée de façon continue et positionnée sur la platine d'un microscope inversé. La chambre est perfusée avec du tampon physiologique contenant (en mM): NaCl 140 ; KCl 5.4 ; $CaCl_2$ 1 ; $MgCl_2$ 0,8 ; HEPES 10 et glucose 5,6 (pH = 7,4 ; 290 mOsmol/kg$H_2O$).

La contraction des myocytes est induite une fois par seconde (1 Hz) avec des électrodes de champ en platine placées dans la chambre et reliées à un stimulateur. Les images sont saisies de façon continue avec un objectif x 20 et transmises à une caméra CCD à raison de 240 échantillons/s. Les images de la caméra CCD sont projetées sur un écran vidéo.

Les myocytes ont été sélectionnés pour l'étude selon le critère suivant : une apparence en forme de baguette avec des striations bien apparentes et pas de vacuole intracellulaire, pas de contraction spontanée quand on les stimule avec 1 mM $Ca^{2+}$, et avec une longueur de repos et une amplitude de contraction constantes. La longueur des sarcomères a été mesurée grâce à un programme d'analyse d'image vidéo et les données ont été saisies à un rythme de 240 échantillons/s. Les images caméra ont été converties en mesures de longueur de sarcomère. Le pourcentage de contraction est calculé à partir de ces données sur la longueur du sarcomère.

**Analyse des données**

Toutes les données ont été exprimées en moyenne ± écart-type. Les comparaisons des données entre les différents groupes ont été réalisées par ANOVA suivi d'un test de Student avec une différence significative à $p<0.05$.

**ðŧ Protocole expérimental**

L'apoptose est induite dans les cardiomyocytes isolés par exposition pendant 3 à 8h à 1 μM de doxorubicine ajoutée dans une solution isotonique contenant (en mM) NaCl 110, KCl 5,4, $Na_2PO_4$ 0,33, $NaHCO_3$ 25, Glucose 5, $MgCl_2$ 0,8, $CaCl_2$ 1, pH ajusté à 7,4. Le marquage à l'annexine V a été réalisé 3 h après le début de l'exposition à la doxorubicine puisque ce phénomène apparaît très tôt dans la cascade apoptotique. Les mesures d'activité caspase-3 sont réalisées 8 h après l'exposition à la doxorubicine puisque ce phénomène a lieu plus tard dans le phénomène d'apoptose. La contractilité des cardiomyocytes a été mesurée toutes les heures pendant les 8 h d'exposition à la doxorubicine. Après tous les traitements, les cellules ont été comparées aux cardiomyocytes contrôles non exposés à la doxorubicine.

Les cardiomyocytes ont été prétraités avec le composé 3,5-seco-4-nor-cholestan-5-one oxime- 3-ol pendant 15 min avant l'exposition à la doxorubicine. Deux concentrations de ce composé ont été testées lors de cette étude : 0,1 et 0,3 μM.

➢ **Résultats**

La longueur moyenne des sarcomères des cellules utilisées dans cette étude n'était pas significativement différente entre les groupes.

◦ Effet de la doxorubicine sur la contractilité des myocytes et l'apoptose

L'exposition à la doxorubicine a résulté en une diminution au cours du temps du raccourcissement du sarcomère. Le raccourcissement du pic sous doxorubicine était similaire au contrôle pendant les trois premières heures puis devint significativement diminué après 4 h d'exposition (-53,20 ± 7,70% contre - 19,49 ± 2,06% par rapport à la ligne de base de la doxorubicine et du contrôle respectivement, $p<0.05$, $n=5$).

Le traitement avec 1 μM de doxorubicine a induit l'apoptose avec une augmentation significative en marquage à l'annexine V et en activité caspase-3.

◦ Effet du 3,5-seco-4-nor-cholestan-5-one oxime-3-ol sur le dysfonctionnement au niveau de la contractilité induite par la doxorubicine et sur l'apoptose.

Le traitement avec 1 μM de doxorubicine a résulté en une réduction significative du raccourcissement du pic des cardiomyocytes ventriculaires qui est abolie en présence de 3,5-seco-4-nor-cholestan-5-one oxime- 3-ol (0,1 et 0,3 μM). En effet, après 4 h d'exposition, le raccourcissement du pic sous doxorubicine (-53,20 ± 7,70%) devint significativement diminué avec le composé à 0,1 μM (-18,9 ± 5,4%) et à 0,3 μM (-8,1 ± 9,6%) par rapport à la ligne de base.

De plus, les augmentations de marquage à l'annexine V et d'activité caspase-3, dues à la doxorubicine ont été bloquées par le 3,5-seco-4-nor-cholestan-5-one oxime- 3-ol à 0,1 et 0,3 μM.

L'apoptose évaluée en % de changement en marquage à l'annexine V 3h après doxorubicine donne les résultats suivants : contrôle : 100% ; doxorubicine : 320%±48,7 ; doxorubicine + 3,5-seco-4-nor-cholestan-5-one oxime- 3-ol 0,1 μM : 116,3%±15,1 ; doxorubicine + 3,5-seco-4-nor-cholestan-5-one oxime- 3-ol 0,3 μM : 137,3%±19,3. Les résultats concernant les mesures d'activité caspase-3 sont les suivants: contrôle : 19±9 fmol/min ; doxorubicine: 120±15 fmol/min ; doxorubicine + 3,5-seco-4-nor-cholestan-5-one oxime- 3-ol 0.1 μM : 27±20 fmol/min ; doxorubicine + 3,5-seco-4-nor-cholestan-5-one oxime- 3-ol 0.3 μM : 15±7 fmol/min.

➢ **Commentaires et conclusions**

Le composé 3,5-seco-4-nor-cholestan-5-one oxime-3-ol montre un effet cardioprotecteur sur le dysfonctionnement de contractilité induit par la doxorubicine et sur l'apoptose sur des cardiomyocytes isolés de lapin. La molécule, quand utilisée à des doses appropriées peut effectivement protéger contre la cardiotoxicité induite par la doxorubicine qui est connue comme étant le facteur limitant dans le traitement de patients cancéreux avec cette anthracycline. Ainsi, le composé 3,5-seco-4-nor-cholestan-5-one oxime-3-ol pourrait être utilisé pour limiter la cardiotoxicité de la doxorubicine chez ces patients.

**Exemple 13 : Effet des composés 3,5-seco-4-nor-cholestan-5-one oxime-3-ol et 3,5-seco-4-nor-cholestan-5-one oxime-3-N,N-diméthylglycine ester dans un modèle *in vivo* d'infarctus du myocarde chez la souris**

Le but de cette expérience est d'étudier *in vivo* dans un modèle d'occlusion-reperfusion coronaire chez la souris, les propriétés cardioprotectrices des composés 3,5-seco-4-nor-cholestan-5-one oxime-3-ol et 3,5-seco-4-nor-cholestan-5-one oxime-3-N,N-dimethylglycine ester.

Les composés à tester sont administrés par voie intraveineuse chez la souris, 5 minutes avant reperfusion du myocarde précédemment ischémié. A titre de contrôles, les véhicules des composés, respectivement la bêta-cyclodextrine (ci-après appelée □CD) et l'eau, sont administrés dans les mêmes conditions que les composés.

Afin de déterminer l'effet des composés testés, on mesure la taille de l'infarctus après une reperfusion de 24 heures.

**Instrumentation chirurgicale des animaux**

Des souris mâles C57BL6 âgées de 6 à 8 semaines sont anesthésiées par du pentobarbital sodique (50 mg/kg i.p.) et sont ventilées après intubation avec un mélange de $O_2/CO_2$ (95%/5%) tout au long de l'expérience. L'électrocardiogramme de surface (ECG) est enregistré et visualisé sur un oscilloscope pendant toute la durée de la chirurgie. Dans des conditions d'asepsie rigoureuses, la veine jugulaire est cathétérisée pour l'administration intraveineuse des composés. Une thoracotomie latérale gauche est effectuée et après péricardiotomie, une branche majeure de l'artère coronaire gauche est repérée dans la région latéro-postérieure du ventricule gauche. Un fil prolène numéro 8 est positionné autour de cette artère de manière à former une boucle mobile pour la réalisation d'une occlusion coronaire temporaire.

**Protocoles expérimentaux**

Toutes les souris ont fait l'objet d'une occlusion coronaire temporaire de 30 minutes. L'ischémie de la région du myocarde est confirmée par la présence d'une cyanose de la surface du myocarde et par une déviation du segment ST de l'électrocardiogramme.
Chacun des composés (groupe traité, n = 10) ou son solvant (groupe contrôle, n = 10) a été administré en bolus par voie intraveineuse 5 min avant la levée des 30 min d'occlusion coronaire.
Le 3,5-seco-4-nor-cholestan-5-one oxime-3-ol, préalablement dissout à une concentration finale de 0,46 mg/ml dans une solution de □CD à 30% dans du tampon phosphate préparée par saturation suivi d'une centrifugation, est administré à la dose de 1 mg/kg.
Le 3,5-seco-4-nor-cholestan-5-one oxime- 3-N,N-dimethylglycine ester, préalablement dissout à une concentration finale de 1,56 mg/ml dans de l'eau stérile par agitation et sonication est administré à la dose de 3,9 mg/kg.
Le même volume de véhicule est administré dans les groupes contrôles correspondants.
La reperfusion a été confirmée par l'aspect du segment QRS de l'électrocardiogramme.
Après fermeture du thorax plan par plan et évacuation du pneumothorax par aspiration à l'aide d'un drain, les souris sont ensuite progressivement réveillées et sevrées de leur assistance ventilatoire jusqu'à reprise d'une ventilation spontanée normale. Si nécessaire, de la buprénorphine (1 mg/kg) est administrée par voie intra-péritonéale pour assurer une couverture antalgique efficace.
Vingt-quatre heures après la fin de l'occlusion coronaire, les souris sont ré-anesthésiées par du pentobarbital sodique (50 mg/kg i.p.) et de l'héparine est administrée par voie intraveineuse (héparine Choay© 1000UI/kg). Une ligature de l'artère coronaire est réalisée au même endroit que celle ayant fait l'objet de la première occlusion 24h auparavant. Les souris sont ensuite euthanasiées par une dose létale de chlorure de potassium saturée et leur coeur est rapidement excisé puis monté par l'artère aorte sur un système de perfusion rétrograde de type Langendorf.
Une solution de bleu Evans à 5 % est perfusée de manière rétrograde afin que le myocarde sain soit coloré en bleu ; la zone rendue ischémique lors de l'occlusion coronaire, ou « aire à risque AR » restant non colorée par défaut.
Le ventricule gauche est ensuite découpé en 5 tranches d'épaisseurs égales (1 mm) à l'aide d'un tranchoir spécialement conçu pour les coeurs de souris (Les Isolants de Paris, Palaiseau) qui sont ensuite pesées.
Ces tranches sont incubées dans une solution de chlorure de triphenyltetrazolium (TTC, Sigma, Poole, UK) à 1 % pH 7, 4 pendant 20 min à 37° C puis fixées dans du formol à 4 %. Le TTC a la propriété de colorer en rouge le myocarde non infarci, et donc de faire apparaître les zones infarcies en blanc. Chaque tranche de coeur est placée sous stéréo-microscope pour une prise de photo numérique de haute définition.
La quantification de l'infarctus et de l'aire à risque est effectuée par planimétrie (Scion Image, Scion, Frederick MD, USA) et rapportée au poids de chaque tranche.
L'aire à risque (zone non-bleue) est exprimée en pourcentage du poids du ventricule gauche. Les tailles d'infarctus sont exprimées en pourcentage du poids de l'aire à risque.
Toutes les valeurs de taille d'infarctus et d'aire à risque sont exprimées en moyenne $\pm$ SEM. Une ANOVA à un facteur suivie d'un test t de Student non pairé avec correction de Bonferonni est utilisée pour comparer les aires à risque et les tailles d'infarctus entre les groupes expérimentaux, le seuil de signification étant fixé à $p<0,05$.

**Résultats et conclusions**

Les tailles d'infarctus des souris traitées avec les composés diffèrent significativement de celles des souris traitées par le véhicule.
Les résultats présentés dans le tableau ci-dessous sont exprimés d'une part par l'aire à risque et d'autre part

par la taille de l'infarctus.

| souris traitées | Aire à risque | Taille de l'infarctus |
|---|---|---|
| 3,5-seco-4-nor-cholestan-5-one oxime-3-ol | 9+/-4% | 21+/-7% |
| Véhicule | 14+/-4% | 33+/-7% |
| | P<0,01 | P<0,0007 |
| réduction de la taille de l'infarctus | | 36,4% |
| 3,5-seco-4-nor-cholestan-5-one oxime- 3-N,N-diméthylglycine ester | 8+/-3% | 23+/-9% |
| Véhicule | 16+/-8% | 40+/-15% |
| | P<0,07 | P<0,006 |
| réduction de la taille de l'infarctus | | 42,5% |

Dans le modèle expérimental utilisé, les deux composés testés réduisent la taille d'infarctus. De plus, les résultats ont montré que les composés réduisent les tailles d'infarctus quelle que soit l'étendue de la zone à risque.

Ces résultats montrent donc que ces deux composés présentent *in vivo* des effets cardioprotecteurs.

**Exemple 14 : Effet du composé 3,5-seco-4-nor-cholestan-5-one oxime-3-ol dans un modèle *in vivo* d'hépatotoxicité aigu.**

Dans cette expérience, on teste la capacité du composé 3,5-seco-4-nor-cholestan-5-one oxime-3-ol à protéger les hépatocytes.

Les hépatocytes comme de nombreuses autres cellules portent le récepteur Fas/CD95 sur leur membrane cytoplasmique. La stimulation de cette voie Fas induit la mort cellulaire en activant la cascade des caspases.

Un modèle aigu de dommage hépatique peut être induit par une injection unique de l'anticorps anti-Fas Jo2 (Ogasawara et al., Nature, août 1993), produisant des dommages hépatiques sévères et ressemblant à l'hépatite virale, autoimmune ou induite par des drogues.

L'Alanine Aminotransferase (ALAT) aussi appelée la Serum Glutamic Pyruvic Transaminase sérique (SGPT) est une enzyme présente dans les hépatocytes. Son activité augmente de façon importante dans le plasma après lyse hépatique et est donc un bon marqueur pour évaluer le dommage hépatique.

**Matériels et Méthodes**

**Animaux**

Des souris adultes CD1 mâles en provenance de "Elevage Janvier", (Le Genest-Saint-Isle, France) ont été utilisées. Les animaux ont été identifiés individuellement et ont eu libre accès à l'eau et à la nourriture.

Les installations ont été maintenues à un cycle contrôlé de lumière (7:00 - 19:00), et à des températures de 20 $\pm$ 2 °C et d'humidité de 50 $\pm$ 20 %.

**Préparation de l'anticorps Jo2**

Une solution stock d'anticorps monoclonal de hamster anti-souris CD95 (Fas), appelé Jo2, provenant de Pharmingen (BD Biosciences, ref. 554254 batch 32699) est préparée à la concentration de 1 mg/mL dans de l'eau. Les dilutions utilisées sont réalisées dans du chlorure de sodium à 0,9% dans de l'eau.

**Préparation du composé à tester**

La quantité désirée de 3,5-seco-4-nor-cholestan-5-one oxime-3-ol est pesée et broyée en une fine poudre, puis mélangée avec du Cremophore EL (Sigma C5135) et de l'éthanol absolu (Carlo Erba RPE 41571) (respectivement 5% et 10% du volume final). Après dissolution complète, du chlorure de sodium à 0,9% dans l'eau est ajouté extemporanément (85% du volume final).

Deux types d'expériences sont conduites : l'intoxication par Jo2 suivi d'un dosage des ALAT et l'intoxication létale par Jo2.

**- intoxication par Jo2 et dosage des ALAT :**

**Protocole**

Un prétraitement par le 3,5-seco-4-nor-cholestan-5-one oxime-3-ol est réalisé aux doses de 10 et 30 mg/kg par administration intrapéritonéale 1h avant l'administration de l'anticorps Jo2. L'anticorps Jo2 est administré par injection intrapéritonéale à la dose de 125 μg/kg dans un volume de 5 mL/kg de poids corporel.

Un contrôle est réalisé sur des animaux recevant un prétraitement par administration intrapéritonéale 1 h avant l'administration de l'anticorps d'un volume identique de solution ayant été utilisée pour la préparation du composé à tester, sans composé.

**Dosage des ALAT**

Du sang des souris anesthésiées est prélevé 24h après administration de Jo2. Le dosage des ALAT est realisé en utilisant un kit (Roche Diagnostics) avec un spectrophotomètre (Hitachi Modular), selon la méthode standardisée par l'IFCC (Fédération Internationale de Chimie Clinique).

**Résultats et conclusions**

L'administration intrapéritonéale de Jo2 à 125 μg/kg n'induit pas de mortalité chez les souris dans les 24h suivant l'injection.

L'activité ALAT est significativement réduite par le composé 3,5-seco-4-nor-cholestan-5-one oxime-3-ol à 10 et 30 mg/kg.

Table 1 : Activité ALAT mesurée 24h après administration de Jo2

| Traitement | Activité ALAT (U/L) Moyenne +/- SEM (n) |
|---|---|
| Contrôle | 2860 ± 382 (61) |
| 3,5-seco-4-nor-cholestan-5-one oxime-3-ol (10 mg/kg) | 511 ± 140 (19) ** |
| 3,5-seco-4-nor-cholestan-5-one oxime-3-ol (30 mg/kg) | 533 ± 150 (20) ** |
| ** : p< 0.01, ANOVA suivi du test comparatif de Dunnett effectué par rapport au groupe placebo | |

Le 3,5-seco-4-nor-cholestan-5-one oxime-3-ol administré à 10 et 30 mg/kg, 1 h avant l'anticorps Jo2, a permis de limiter la mort cellulaire induite par une dose sublétale d'anticorps.

L'activité ALAT, biomarqueur de la cytolyse hépatique dans le plasma, est significativement plus basse chez les souris traitées par le 3,5-seco-4-nor-cholestan-5-one oxime-3-ol que chez les souris contrôles non traitées.

- **Intoxication létale par l'anticorps Jo2**

Dans cette expérience on évalue l'effet du 3,5-seco-4-nor-cholestan-5-one oxime-3-ol sur la survie des animaux après administration d'une dose létale de l'anticorps Jo2.

**Protocole**

L'anticorps Jo2 est administré par injection intrapéritonéale à la dose de 200 ou 250 μg/kg dans un volume de 5 mL/kg de poids corporel.

Le 3,5-seco-4-nor-cholestan-5-one oxime-3-ol est testé à 10 et 30 mg/kg, en prétraitement, 1 h avant l'administration de Jo2 ou en post-traitement, 1 h après l'administration de Jo2.

Un contrôle est réalisé sur des animaux recevant un prétraitement par administration intrapéritonéale 1 h avant ou 1 h après l'administration de l'anticorps d'un volume identique de solution ayant été utilisée pour la préparation du composé à tester, sans composé.

**Résultats et conclusions**

Table 2 : Survie des animaux à 24h

| | Dose Jo2 | Groupe | Mortalité (24h) / nombre total d'animaux | Survie (%) |
|---|---|---|---|---|
| Expérience 1 | 250 μg/kg | Contrôle | 15/20 | 25 |
| | | Avec prétraitement à 10 mg/kg | 7/20 | 65 |
| Expérience 2 | 250 μg/kg | Contrôle | 18/20 | 10 |
| | | Avec prétraitement à 30 mg/kg | 2/20 | 90 |
| Expérience 3 | 250 μg/kg | Contrôle | 18/20 | 10 |
| | | Avec post-traitement à 10 mg/kg | 17/20 | 15 |
| Expérience 4 | 250 μg/kg | Contrôle | 19/20 | 5 |
| | | Avec post-traitement à 30 mg/kg | 13/20 | 35 |
| Expérience 5 | 200 μg/kg | Contrôle | 14/20 | 30 |
| | | Avec post-traitement à 10 mg/kg | 8/20 | 60 |
| Expérience 6 | 200 μg/kg | Contrôle | 16/20 | 20 |
| | | Avec post-traitement à 30 mg/kg | 8/20 | 60 |

Aux doses administrées, l'anticorps Jo2 induit une importante mortalité en 24h (70 à 100% des animaux) dans le groupe contrôle.

Le prétraitement et/ou le post-traitement au 3,5-seco-4-nor-cholestan-5-one oxime-3-ol aux doses administrées induit une augmentation de la survie des animaux.

Ainsi, lorsqu'une dose létale d'anticorps est utilisée (200 ou 250 μg/kg), le 3,5-seco-4-nor-cholestan-5-one oxime-3-ol, administré à 10 et 30 mg/kg, 1 h avant ou après l'anticorps, augmente la survie des souris sur 24h.

## Conclusions

Le modèle d'hépatotoxicité aigüe induite chez la souris par un anticorps anti Fas (Jo2) a permis de mettre en évidence les propriétés hépatoprotectrices du 3,5-seco-4-nor-cholestan-5-one oxime-3-ol.

Ces effets remarquables permettent d'envisager pour les composés de formule I une utilisation dans la préparation d'un médicament cytoprotecteur en général.

## Revendications

1. Utilisation d'un composé répondant à la formule I

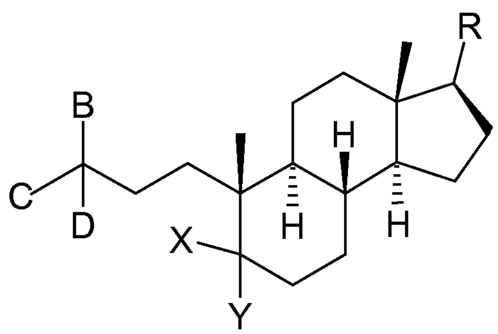

(I)

dans laquelle

- X et Y pris ensemble représentent une fonction céto (=O), un groupement oxime (=NOH) ou un groupement

méthyloxime (=NOMe) ou X représente un hydroxyle et Y un atome d'hydrogène ;
- B représente un radical hydroxyle et C et D, identiques ou différents, représentent un atome d'hydrogène, ou un radical alkyle, linéaire ou ramifié, comprenant de 1 à 4 atomes de carbone ; ou
B et C pris ensemble représentent une fonction céto et D un radical méthyle, hydroxyle, ou méthylamine ; ou
B et C représentent un atome d'hydrogène et D un radical méthylamine ; ou
B et C pris ensemble représentent un groupement oxime et D un radical méthyle ; et
- R représente un radical alkyle, linéaire ou ramifié, comprenant de 1 à 10 atomes de carbone ;

ou l'un de ses sels d'addition avec les acides pharmaceutiquement acceptables, ou l'un de ses esters ou l'un des sels d'addition avec les acides pharmaceutiquement acceptables desdits esters, pour la préparation d'un médicament cytoprotecteur contre les conséquences au niveau cellulaire de l'ostéoporose, l'ostéomyélite, les arthrites, l'ostéoarthrite, l'arthrite rhumatoïde et l'arthrite psoriatique, la nécrose avasculaire, la fibrodysplasie ossifiante progressive, le rachitisme, le syndrome de Cushing ; la dystrophie musculaire de Duchenne, les dystrophies myotoniques, les myopathies et les myasthénies ; les dermatites, l'eczéma, le psoriasis, l'ischémie cardiaque, l'infarctus du myocarde, la cardiopathie ischémique, l'insuffisance cardiaque chronique ou aigue, la dysrythmie cardiaque, la fibrillation auriculaire, la fibrillation ventriculaire, la tachycardie paroxystique, l'insuffisance cardiaque, l'anoxie, l'hypoxie, les effets secondaires dus à des thérapies avec des agents anticancéreux ; l'athérosclérose, les scléroses artérielles, les maladies vasculaires périphériques, les anévrismes ; l'anémie, l'amyloïdose vasculaire, les hémorragies, la drépanocytose, le syndrome de la fragmentation des globules rouges, la neutropénie, la leucopénie, l'aplasie médullaire, la pancytopénie, la thrombocytopénie, l'hémophilie ; la pneumonie, l'asthme ; les maladies chroniques obstructives des poumons comme par exemple les bronchites chroniques et l'emphysème ; les ulcères du tractus gastro-intestinal ; les hépatites particulièrement les hépatites d'origine virale ou ayant pour agent causal d'autres agents infectieux, les hépatites autommunes, les hépatites fulminantes, la maladie de Wilson, les cirrhoses, la stéatose hépatique non alcoolique, les maladies du foie dues à des toxines ou des médicaments ; les pancréatites aigues ou chroniques ; les diabètes mellitus et insipide, les thyroïdites ; les désordres rénaux aigus ou la glomérulonéphrite ; la septicémie ; les intoxications sévères par des agents chimiques, des toxines ou des médicaments ; le syndrome du vieillissement accéléré ; la maladie de Crohn, la polyarthrite rhumatoïde ; le lupus érythémateux ; les périodontites ; l'otosclérose, la surdité induite par des antibiotiques ; la dystrophie musculaire congénitale avec anomalie mitochondriale structurelle, le syndrome des MELAS, le syndrome de MERFF, le syndrome de Pearson, le syndrome de MIDD, le syndrome de Wolfram, la dystonie ;
à l'exception de la préparation d'un médicament neuroprotecteur.

2. Utilisation selon la revendication 1, **caractérisée en ce que** dans la formule I, R représente le radical du cholestane de formule II

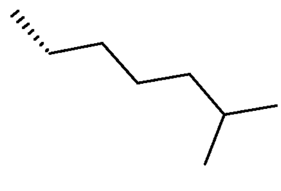

(II)

3. Utilisation selon l'une quelconque des revendications 1 ou 2, **caractérisée en ce que** dans la formule I, X et Y pris ensemble représentent une fonction céto.

4. Utilisation selon l'une quelconque des revendications 1 à 3, **caractérisée en ce que** dans la formule I, B représente un radical hydroxyle et C et D, identiques ou différents, représentent un atome d'hydrogène ou, un radical alkyle, linéaire ou ramifié, comprenant de 1 à 4 atomes de carbone.

5. Utilisation selon l'une quelconque des revendications 1 à 3, **caractérisée en ce que** dans la formule I, B et C pris ensemble représentent une fonction céto et D représente un radical méthyle.

6. Utilisation selon l'une quelconque des revendications 1 ou 2, **caractérisée en ce que** dans la formule I, X et Y pris ensemble représentent un groupement oxime.

7. Utilisation selon la revendication 1, **caractérisée en ce qu'**il est choisi parmi
le 3,5-seco-4-nor-cholestan-5-one oxime- 3-ol,

le 3,5-seco-4-nor-cholestan-5-one oxime-3-méthyl alcool, ou
le 3,5-seco-4-nor-cholestan-5-one oxime-3-diméthyl alcool,
ou l'un de ses sels d'addition avec les acides pharmaceutiquement acceptables, ou l'un de ses esters ou l'un des sels d'addition avec les acides pharmaceutiquement acceptables desdits esters.

## Patentansprüche

1. Verwendung einer Verbindung gemäß Formel I

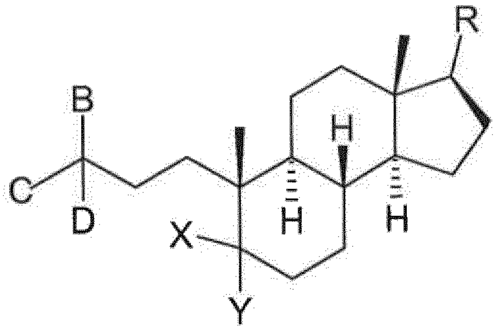

(I)

wobei

- X und Y zusammen genommen eine Ketofunktion (=O), eine Oxim-Gruppe (=NOH) oder eine Methyloxim-Gruppe (=NOMe) repräsentieren oder X ein Hydroxyl repräsentiert und Y ein Wasserstoffatom repräsentiert;
- B einen Hydroxylrest repräsentiert und C und D, identisch oder unterschiedlich, ein Wasserstoffatom oder einen Alkylrest, linear oder verzweigt, mit 1 bis 4 Kohlenstoffatomen repräsentieren; oder
B und C zusammen genommen eine Ketofunktion repräsentieren und D einen Methyl-, Hydroxyl- oder Methylaminrest repräsentiert; oder
B und C ein Wasserstoffatom repräsentieren und D einen Methylaminrest repräsentiert; oder
B und C zusammen genommen eine Oximgruppe repräsentieren und D einen Methylrest repräsentiert; und
- R einen Alkylrest, linear oder verzweigt, mit 1 bis 10 Kohlenstoffatomen repräsentiert;

oder eines ihrer Additionssalze mit den pharmazeutisch akzeptablen Säuren oder einen ihrer Ester oder eines ihrer Additionssalze mit den pharmazeutisch akzeptablen Säuren der genannten Ester, für die Herstellung eines Zellschutzmedikaments gegen die zellulären Folgen von Osteoporose, Osteomyelitis, einer Arthritis, Osteoarthritis, rheumatoide Arthritis und psoriatische Arthritis, avaskuläre Nekrose, Fibrodysplasia ossificans progressiva, Rachitis, Cushing-Syndrom; Duchenne-Muskeldystrophie, myotone Dystrophien, Myopathien und Myasthenien; eine Dermatitis, Ekzem, Psoriase, Herzischämie, Myokardinfarkt, ischämische Herzkrankheit, chronische oder akute Herzinsuffizienz, Herzrhythmusstörung, aurikuläre Fibrillation, ventrikuläre Fibrillation, paroxystische Tachykardie, Herzinsuffizienz, Anoxie, Hypoxie, Nebenwirkungen aufgrund von Therapien mit Antikrebsmitteln; Atherosklerose, arterielle Sklerosen, periphere Gefäßkrankheiten, Aneurismen; Anämie, Gefäßamyloidose, Hämorrhagien, Sichelzellanämie, Syndrom der Fragmentation von roten Blutkörperchen, Neutropenie, Leukopenie, Myelosuppression, Panzytopenie, Thrombozytopenie, Hämophilie; Pneumonie, Asthma; chronische obstruktive Lungenerkrankungen wie zum Beispiel chronische Bronchitis und Emphysem; Geschwüre des Magen-Darm-Trakts; Hepatitis, insbesondere Hepatitis viralen Ursprungs oder durch andere Infektionsmittel verursacht, autoimmune Hepatitis, fulminante Hepatitis, Wilson-Krankheit, Zirrhosen, nichtalkoholische Fettleber, Krankheiten der Leber aufgrund von Toxinen oder Medikamenten; akute oder chronische Pankreatitis; Diabetes mellitus und insipidus, Thyroidite; akute Nierenstörungen oder Glomerulonephrititis; Sepsis; ernsthafte Intoxikationen durch chemische Mittel, Toxine oder Medikamente; beschleunigtes Alterungssyndrom; Crohn-Krankheit, rheumatoide Polyarthritis; Lupus erythematodes; eine Periodontitis; Otosklerose, durch Antibiotika verursachte Gehörlosigkeit; kongenitale Muskeldystrophie mit struktureller mitochondrialer Anomalie, MELAS-Syndrom, MERFF-Syndrom, Pearson-Syndrom, MIDD-Syndrom, Wolfram-Syndrom, Dystonie;
mit Ausnahme der Herstellung eines Neuroprotektionsmedikaments.

2. Verwendung nach Anspruch 1, **dadurch gekennzeichnet, dass** in Formel I R den Rest von Cholestan von Formel

II repräsentiert:

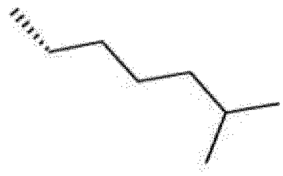

(II)

3. Verwendung nach Anspruch 1 oder 2, **dadurch gekennzeichnet, dass** in Formel I X und Y, zusammen genommen, eine Ketofunktion repräsentieren.

4. Verwendung nach einem der Ansprüche 1 bis 3, **dadurch gekennzeichnet, dass** in Formel I B einen Hydroxylrest repräsentiert und C und D, identisch oder unterschiedlich, ein Wasserstoffatom oder einen Alkylrest, linear oder verzweigt, mit 1 bis 4 Kohlenstoffatomen repräsentieren.

5. Verwendung nach einem der Ansprüche 1 bis 3, **dadurch gekennzeichnet, dass** in Formel I B und C, zusammen genommen, eine Ketofunktion repräsentieren und D einen Methylrest repräsentiert.

6. Verwendung nach Anspruch 1 oder 2, **dadurch gekennzeichnet, dass** in Formel I X und Y, zusammen genommen, eine Oxim-Gruppe repräsentieren.

7. Verwendung nach Anspruch 1, **dadurch gekennzeichnet, dass** sie ausgewählt ist aus
3,5-Seco-4-nor-cholestan-5-on Oxim-3-ol,
3,5-Seco-4-nor-cholestan-5-on Oxim-3-methylalkohol, oder 3,5-Seco-4-nor-cholestan-5-on Oxim-3-dimethylalkohol,
oder einem ihrer Additionssalze mit pharmazeutisch akzeptablen Säuren oder einem ihrer Ester oder einem ihrer Additionssalze mit pharmazeutisch akzeptablen Säuren der genannten Ester.

**Claims**

1. Use of a compound corresponding to Formula I

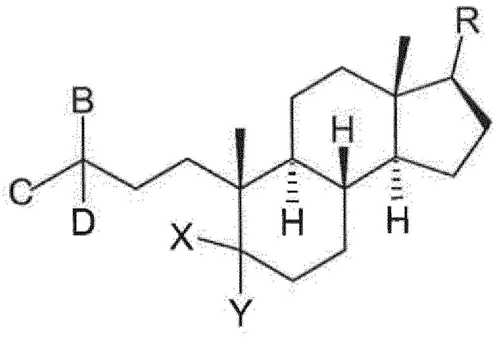

(I)

in which

- X and Y together represent a keto function (=O), an oxime group (=NOH) or a methyloxime group (=NOMe) or X represents a hydroxyl and Y a hydrogen atom;
- B represents a hydroxyl radical and C and D, identical or different, represent a hydrogen atom, or a linear or branched alkyl radical, comprising from 1 to 4 carbon atoms; or
B and C together represent a keto function and D a methyl, hydroxyl, or methylamine radical; or
B and C represent a hydrogen atom and D a methylamine radical; or
B and C together represent an oxime group and D a methyl radical; and
- R represents a linear or branched alkyl radical, comprising from 1 to 10 carbon atoms;

or one of the addition salts thereof with pharmaceutically acceptable acids, or one of the esters thereof or one of the addition salts with pharmaceutically acceptable acids of said esters, for the preparation of a cytoprotective medicament against the consequences at cellular level of osteoporosis, osteomyelitis, arthritis, osteoarthritis, rheumatoid arthritis and psoriatic arthritis, avascular necrosis, progressive ossifying fibrodysplasia, rickets, Cushing syndrome; Duchenne muscular dystrophy, myotonic dystrophies, myopathies and myasthenias; dermatitis, eczema, psoriasis, cardiac ischemia, myocardial infarction, ischemic heart disease, chronic or acute cardiac insufficiency, cardiac dysrhythmia, atrial fibrillation, ventricular fibrillation, paroxysmal tachycardia, cardiac insufficiency, anoxia, hypoxia, secondary effects due to treatment with antineoplastic agents; artherosclerosis, arterial sclerosis, peripheral vascular diseases, aneurysms; anemia, vascular amyloidosis, hemorrhage, sickle-cell anemia, red cell fragmentation syndrome, neutropenia, leucopenia, medullar aplasia, pancytopenia, thrombocytopenia, hemophilia; pneumonia, asthma; chronic obstructive pulmonary diseases such as for example chronic bronchitis and emphysema; ulcers of the gastrointestinal tract; hepatitis, particularly hepatitis of viral origin or having other infectious agents as causal agent, autoimmune hepatitis, fulminant hepatitis, Wilson's disease, cirrhosis, non-alcoholic hepatic steatosis, diseases of the liver due to toxins or drugs; acute or chronic pancreatitis; diabetes mellitus and diabetes insipidus, thyroiditis; acute renal disorders or glomerulonephritis; septicemia; severe intoxication by chemical agents, toxins or drugs; accelerated aging syndrome; Crohn's disease, rheumatoid polyarthritis; lupus erythematosus; parodontitis; otosclerosis, antibiotic-induced deafness; congenital muscular dystrophy with structural mitochondrial anomaly, MELAS syndrome, MERFF syndrome, Pearson syndrome, MIDD syndrome, Wolfram syndrome, dystonia;
with the exception of the preparation of a neuroprotective medicament.

2. Use according to claim 1, **characterized in that** in Formula I, R represents the radical of the cholestane of formula II

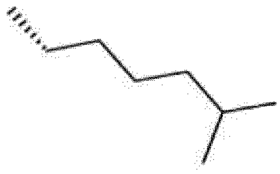

(II)

3. Use according to any one of claims 1 or 2, **characterized in that** in Formula I, X and Y together represent a keto function.

4. Use according to any one of claims 1 to 3, **characterized in that** in Formula I, B represents a hydroxyl radical and C and D, identical or different, represent a hydrogen atom or, a linear or branched alkyl radical, comprising from 1 to 4 carbon atoms.

5. Use according to any one of claims 1 to 3, **characterized in that** in Formula I, B and C together represent a keto function and D represents a methyl radical.

6. Use according to any one of claims 1 or 2, **characterized in that** in Formula I, X and Y together represent an oxime group.

7. Use according to claim 1, **characterized in that** it is chosen from
3,5-seco-4-nor-cholestan-5-one oxime- 3-ol,
3,5-seco-4-nor-cholestan-5-one oxime-3-methyl alcohol, or
3,5-seco-4-nor-cholestan-5-one oxime-3-dimethyl alcohol,
or one of the addition salts thereof with pharmaceutically acceptable acids, or one of the esters thereof or one of the addition salts with pharmaceutically acceptable acids of said esters.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2005007192 A2
- WO 2004106370 A
- WO 2006027454 A


### Littérature non-brevet citée dans la description


- **SMITH.** Steroid lysis of protoplasts and effects of stabilizers and steroid antagonists. *APPLIED MICROBIOLOGY,* 1965, vol. 13 (5), ISSN 0003-6919, 706-12
- **RODEWALD, W. J. ; WICHA, J.** Azasteroidal alkaloids. Synthesis of A-nor-B-homo-5-azacholestane. *Bulletin de l'Académie Polonaise des Sciences, Série des Sciences Chimiques,* 1963, vol. 11 (8), 437-441
- **A. D'ANGLEMONT DE TASSIGNY et al.** *Fund Clin Pharmacol,* 2004, vol. 18, 531-38
- **OGASAWARA et al.** *Nature,* Août 1993